(19) Europäisches Patentamt
European Patent Office
Office européen des brevets

(11) EP 4 768 598 A1

(12) EUROPEAN PATENT APPLICATION
published in accordance with Art. 153(4) EPC

(43) Date of publication:
**01.07.2026 Bulletin 2026/27**

(21) Application number: **24877244.4**

(22) Date of filing: **10.10.2024**

(51) International Patent Classification (IPC):
***C12Q 1/6844*** *(2018.01)* ***C40B 40/02*** *(2006.01)*

(52) Cooperative Patent Classification (CPC):
**C12Q 1/6844; C40B 40/02**

(86) International application number:
**PCT/JP2024/036319**

(87) International publication number:
**WO 2025/079650 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.10.2023 PCT/JP2023/036907**

(71) Applicant: **Chugai Seiyaku Kabushiki Kaisha**
**Kita-ku**
**Tokyo 115-8543 (JP)**

(72) Inventors:
- **OKITSU, Koyo**
  **Yokohama City, Kanagawa 2448602 (JP)**
- **IIDA, Takeo**
  **Yokohama City, Kanagawa 2448602 (JP)**
- **ASAWA, Yasunobu**
  **Yokohama City, Kanagawa 2448602 (JP)**
- **MATSUO, Atsushi**
  **Yokohama City, Kanagawa 2448602 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) METHOD FOR SCREENING FOR PEPTIDE USING MULTIPLE LIBRARIES

(57) The present invention provides a method for screening for a candidate peptide capable of binding to a target molecule, the method including the steps of: (1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system; (2) mixing the plurality of nucleic acid display libraries to prepare a mixed nucleic acid display library; (3) bringing the mixed nucleic acid display library into contact with the target molecule; and (4) amplifying a nucleic acid corresponding to the nucleic acid moiety of the barcoded peptide-nucleic acid complex bound to the target molecule, using a barcode primer.

EP 4 768 598 A1

## Description

[Technical Field]

The present invention relates to a method for screening for a peptide using a plurality of libraries.

[Background Art]

mRNA display techniques using a reconstituted cell-free translation system of E. coli have been used for synthesizing a peptide library containing unnatural amino acids and screening for peptides with high binding capacity using the same (Patent Literature 1).

To efficiently find useful peptides in screening, the diversity of the library is very important. For this, it is important to increase the diversity of constituent units (amino acids) of peptides contained in the library. However, when the peptide is synthesized by translation, there is a limit to the types of amino acids that can be incorporated into one codon table, so that there is also a limit to the number of amino acids available. Thus, the creation of a highly diverse library using different cell-free translation systems is being studied (Non Patent Literatures 1 and 2).

[Citation List]

[Patent Literature]

[Patent Literature 1] International Publication No. WO 2013/100132

[Non Patent Literature]

[Non Patent Literature 1] D.E. Hacker et al. ACS Chem. Biol., 2017, 12, 795-804.
[Non Patent Literature 2] M.E. Brousseau et al. Cell Chem. Biol., 2022, 29, 249-258.

[Summary of Invention]

[Technical Problem]

Theoretically, when peptide-nucleic acid complex libraries in which phenotypes and genotypes are associated are separately generated using a plurality of cell-free translation systems, the diversity of peptides, that are phenotypes, can be increased as a whole. However, in this case, since the genotypes are common among the libraries, it is necessary to treat libraries independently depending on each cell-free translation system, and thus the time and effort required for panning increase according to the number of libraries, which is a problem. Thus, it is conceivable to use a method of mixing libraries and performing panning to select more easily candidate peptides that can bind to a target molecule. However, when libraries are mixed and panned against the target molecule, the association between the genotype (nucleic acid) and the phenotype (peptide) is failed, and it is difficult to identify which library the enriched peptide (to be precise, the peptide that is a phenotype corresponding to the enriched nucleic acid) is derived from. In addition, when libraries are mixed and panned, if there is a bias such as an enrichment of peptides that easily bind to the target molecule in a specific library, peptides derived from the library are easy to enrich while peptides derived from other libraries are relatively difficult to enrich (resulting in low enrichment), and thus it may be difficult to obtain hit peptides with various structures.

The present invention has been made considering such circumstances, and an object of the present invention is to provide a method for efficiently screening a plurality of peptide-nucleic acid complex libraries.

[Solution to Problem]

The present inventors have intensively studied to solve the above problems, and as a result, have found a method capable of identifying which library a peptide bound to a target molecule is derived from, even when a plurality of libraries are mixed, by imparting a nucleic acid barcode to a peptide-nucleic acid complex contained in the libraries (barcoding), and finally completed the present invention.

The present invention provides the following [A1] to [A67], [B1] to [B3], [C1] to [C5], or [D1] to [D9].

[A1] A method for screening for a candidate peptide capable of binding to a target molecule, the method comprising the

steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule; and
(4) amplifying a nucleic acid corresponding to the nucleic acid moiety of the barcoded peptide-nucleic acid complex bound to the target molecule, using a barcode primer.

[A2] The method according to [A1], wherein the step (1) includes independently translating a plurality of nucleic acid libraries to prepare a plurality of peptide-nucleic acid complexes using a cell-free translation system.
[A3] The method according to [A1] or [A2], wherein the step (1) includes translating a nucleic acid encoding a peptide to prepare a peptide and linking the peptide to the nucleic acid to prepare a peptide-nucleic acid complex.
[A4] The method according to any of [A1] to [A3], wherein the step (1) includes barcoding the peptide-nucleic acid complex to prepare a barcoded peptide-nucleic acid complex.
[A5] The method according to any of [A1] to [A4], wherein the barcoding is reverse transcribing the nucleic acid moiety of the peptide-nucleic acid complex.
[A6] The method according to any of [A1] to [A5], wherein the barcoding is reverse transcribing the nucleic acid moiety of the peptide-nucleic acid complex with a first primer containing a barcode sequence.
[A7] The method according to any of [A1] to [A6], wherein the plurality of nucleic acid display libraries have different barcode sequences for each nucleic acid display library.
[A8] The method according to any of [A1] to [A7], wherein the barcoded peptide-nucleic acid complexes contained in the plurality of nucleic acid display libraries have different barcode sequences for each nucleic acid display library.
[A9] The method according to any of [A1] to [A8], wherein the plurality of nucleic acid display libraries are nucleic acid display libraries, each of which is independently produced by translation using a different cell-free translation system from one another.
[A10] The method according to any of [A1] to [A9], wherein the plurality of nucleic acid display libraries are nucleic acid display libraries, each of which is produced by translation based on a different genetic code table from one another.
[A11] The method according to any of [A1] to [A10], wherein correspondence between at least one codon and an amino acid is different among the plurality of nucleic acid display libraries.
[A11.1] The method according to any of [A1] to [A10], wherein correspondence between two or more codons and amino acids is different among the plurality of nucleic acid display libraries.
[A11.2] The method according to any of [A1] to [A10], wherein correspondence between three or more codons and amino acids is different among the plurality of nucleic acid display libraries.
[A11.3] The method according to any of [A1] to [A10], wherein correspondence between four or more codons and amino acids is different among the plurality of nucleic acid display libraries.
[A11.4] The method according to any of [A1] to [A10], wherein correspondence between five or more codons and amino acids is different among the plurality of nucleic acid display libraries.
[A11.5] The method according to any of [A1] to [A10], wherein correspondence between 10 or more codons and amino acids is different among the plurality of nucleic acid display libraries.
[A11.6] The method according to any of [A1] to [A10], wherein correspondence between 15 or more codons and amino acids is different among the plurality of nucleic acid display libraries.
[A11.7] The method according to any of [A1] to [A10], wherein correspondence between 1 or more and 48 or less codons and amino acids is different among the plurality of nucleic acid display libraries.
[A11.8] The method according to any of [A1] to [A10], wherein correspondence between 1 or more and 30 or less codons and amino acids is different among the plurality of nucleic acid display libraries.
[Al 1.9] The method according to any of [A1] to [A10], wherein correspondence between 2 or more and 30 or less codons and amino acids is different among the plurality of nucleic acid display libraries.
[A12] The method according to any of [A1] to [A11.9], wherein in the step (2), two or more nucleic acid display libraries are mixed.
[A12.01] The method according to any of [A1] to [A11.9], wherein in the step (2), three or more nucleic acid display libraries are mixed.
[A12.02] The method according to any of [A1] to [A11.9], wherein in the step (2), four or more nucleic acid display libraries are mixed.
[A12.03] The method according to any of [A1] to [A11.9], wherein in the step (2), 2 or more and 400 or less nucleic acid

display libraries are mixed.

[A12.04] The method according to any of [A1] to [A11.9], wherein in the step (2), 2 or more and 200 or less nucleic acid display libraries are mixed.

[A12.05] The method according to any of [A1] to [A11.9], wherein in the step (2), 2 or more and 100 or less nucleic acid display libraries are mixed.

[A12.06] The method according to any of [A1] to [A11.9], wherein in the step (2), 2 or more and 50 or less nucleic acid display libraries are mixed.

[A12.07] The method according to any of [A1] to [A11.9], wherein in the step (2), 2 or more and 15 or less nucleic acid display libraries are mixed.

[A12.08] The method according to any of [A1] to [A11.9], wherein in the step (2), 2 or more and 10 or less nucleic acid display libraries are mixed.

[A12.09] The method according to any of [A1] to [A11.9], wherein in the step (2), 3 or more and 50 or less nucleic acid display libraries are mixed.

[A12.10] The method according to any of [A1] to [A11.9], wherein in the step (2), 4 or more and 50 or less nucleic acid display libraries are mixed.

[A13] The method according to any of [A1] to [A12.1], wherein the barcoded peptide-nucleic acid complex is barcoded so that a library from which the barcoded peptide-nucleic acid complex is derived can be identified.

[A14] The method according to [A13], wherein the "library from which the barcoded peptide-nucleic acid complex is derived" is any of the plurality of nucleic acid display libraries.

[A15] The method according to [A13] or [A14], wherein the barcode sequence is different for each nucleic acid display library so that from which nucleic acid display library the barcoded peptide-nucleic acid complex containing the barcode sequence is derived can be identified.

[A16] The method according to any of [A1] to [A15], wherein the peptide-nucleic acid complex contains a nucleic acid moiety and a peptide moiety, and the nucleic acid moiety contains a nucleic acid sequence encoding the peptide moiety.

[A17] The method according to any of [A1] to [A16], wherein the nucleic acid sequence of a nucleic acid encoding the peptide moiety contains a random sequence.

[A18] The method according to [A17], wherein at least one codon specifying an unnatural amino acid is contained in the random sequence.

[A19] The method according to [A17] or [A18], wherein the random sequence contains at least two triplets of one or more types selected from a plurality of types of triplets.

[A19.1] The method according to [A17] or [A18], wherein the random sequence contains 2 to 1000 triplets of one or more types selected from a plurality of types of triplets.

[A19.2] The method according to [A17] or [A18], wherein the random sequence contains 2 to 500 triplets of one or more types selected from a plurality of types of triplets.

[A19.3] The method according to [A17] or [A18], wherein the random sequence contains 2 to 100 triplets of one or more types selected from a plurality of types of triplets.

[A19.4] The method according to [A17] or [A18], wherein the random sequence contains 2 to 20 triplets of one or more types selected from a plurality of types of triplets.

[A19.5] The method according to [A17] or [A18], wherein the random sequence contains 3 to 18 triplets of one or more types selected from a plurality of types of triplets.

[A19.6] The method according to [A17] or [A18], wherein the random sequence contains 4 to 18 triplets of one or more types selected from a plurality of types of triplets.

[A19.7] The method according to [A17] or [A18], wherein the random sequence contains 4 to 15 triplets of one or more types selected from a plurality of types of triplets.

[A19.8] The method according to [A17] or [A18], wherein the random sequence contains 5 to 15 triplets of one or more types selected from a plurality of types of triplets.

[A19.9] The method according to [A17] or [A18], wherein the random sequence contains 5 to 12 triplets of one or more types selected from a plurality of types of triplets.

[A20] The method according to any of [A19] to [A19.9], wherein the plurality of types of triplets is 64 or less types of triplets.

[A21] The method according to any of [A19] to [A19.9], wherein the one or more types of triplets are randomly selected from the plurality of types of triplets.

[A22] The method according to any of [A1] to [A21], wherein the peptide-nucleic acid complex is a cyclic peptide-nucleic acid complex.

[A23] The method according to any of [A1] to [A22], wherein the peptide-nucleic acid complex is selected from the group consisting of a peptide-DNA complex and a peptide-mRNA complex.

[A24] The method according to any of [A1] to [A23], wherein the peptide-nucleic acid complex is a peptide-mRNA

complex.

[A25] The method according to any of [A1] to [A24], wherein the peptide-nucleic acid complex is a cyclic peptide-mRNA complex.

[A26] The method according to any of [A1] to [A25], further comprising reverse transcribing the nucleic acid moiety of the peptide-nucleic acid complex to obtain the barcoded peptide-nucleic acid complex.

[A27] The method according to any of [A1] to [A26], comprising reverse transcribing using a first primer containing a barcode sequence to obtain the barcoded peptide-nucleic acid complex.

[A28] The method according to any of [A1] to [A27], comprising reverse transcribing the nucleic acid moiety of the peptide-nucleic acid complex using a first primer containing a barcode sequence to obtain the barcoded peptide-nucleic acid complex.

[A29] The method according to any of [A1] to [A28], wherein the barcoded peptide-nucleic acid complex is a barcoded cyclic peptide-nucleic acid complex.

[A30] The method according to any of [A1] to [A29], wherein the nucleic acid moiety of the barcoded peptide-nucleic acid complex contains a barcode sequence and a nucleic acid sequence encoding the peptide.

[A31] The method according to any of [A1] to [A30], wherein the nucleic acid moiety of the barcoded peptide-nucleic acid complex is selected from the group consisting of DNA/mRNA duplex and DNA/DNA duplex.

[A32] The method according to any of [A1] to [A31], wherein the nucleic acid moiety of the barcoded peptide-nucleic acid complex is DNA/mRNA duplex.

[A33] The method according to [A32], wherein the DNA/mRNA duplex is a cDNA/mRNA duplex.

[A34] The method according to [A6] or [A27], wherein the first primer contains an identification barcode sequence.

[A34.1] The method according to [A6] or [A27], wherein the first primer contains a common barcode sequence.

[A34.2] The method according to [A6] or [A27], wherein the first primer contains an identification barcode sequence and a common barcode sequence.

[A34.3] The method according to [A6], [A27], [A34], or [A34.2], wherein the first primer contains an identification barcode sequence having 15-30 bases.

[A34.4] The method according to [A6], [A27], [A34.1], or [A34.2], wherein the first primer contains a common barcode sequence having 15-30 bases.

[A34.5] The method according to [A6] or [A27], wherein the first primer contains a barcode sequence having 25-150 bases.

[A35] The method according to any of [A1] to [A34.5], wherein the cell-free translation system is a reconstituted cell-free translation system.

[A36] The method according to any of [A1] to [A35], wherein the cell-free translation system contains a different combination of amino acids from one another among the plurality of nucleic acid display libraries.

[A37] The method according to any of [A1] to [A36], wherein a nucleic acid sequence of a nucleic acid contained in the nucleic acid library contains a random sequence.

[A38] The method according to [A37], wherein the random sequence contains at least two triplets of one or more types selected from a plurality of types of triplets.

[A38.1] The method according to [A37], wherein the random sequence contains 2 to 1000 triplets of one or more types selected from a plurality of types of triplets.

[A38.2] The method according to [A37], wherein the random sequence contains 2 to 500 triplets of one or more types selected from a plurality of types of triplets.

[A38.3] The method according to [A37], wherein the random sequence contains 2 to 100 triplets of one or more types selected from a plurality of types of triplets.

[A38.4] The method according to [A37], wherein the random sequence contains 2 to 20 triplets of one or more types selected from a plurality of types of triplets.

[A38.5] The method according to [A37], wherein the random sequence contains 3 to 18 triplets of one or more types selected from a plurality of types of triplets.

[A38.6] The method according to [A37], wherein the random sequence contains 4 to 18 triplets of one or more types selected from a plurality of types of triplets.

[A38.7] The method according to [A37], wherein the random sequence contains 4 to 15 triplets of one or more types selected from a plurality of types of triplets.

[A38.8] The method according to [A37], wherein the random sequence contains 5 to 15 triplets of one or more types selected from a plurality of types of triplets.

[A38.9] The method according to [A37], wherein the random sequence contains 5 to 12 triplets of one or more types selected from a plurality of types of triplets.

[A39] The method according to any of [A38] to [A38.9], wherein the plurality of types of triplets is 64 or less types of triplets.

[A40] The method according to any of [A38] to [A39], wherein the one or more types of triplets are randomly selected

from the plurality of types of triplets.

[A41] The method according to any of [A1] to [A40], wherein the nucleic acid library is an mRNA library.

[A41-1] The method according to any of [A1] to [A40], wherein the nucleic acid library contains nucleic acids with different numbers of repeats.

[A41-2] The method according to any of [A1] to [A40], wherein the nucleic acid library contains nucleic acids with the same number of repeats.

[A42] The method according to any of [A1] to [A41], wherein the peptide moiety of the peptide-nucleic acid complex contains a predetermined amino acid, and the nucleic acid moiety contains a triplet encoding the amino acid.

[A43] The method according to any of [A1] to [A42], comprising, between the steps (3) and (4), a step (3A) of eluting the nucleic acid moiety from the barcoded peptide-nucleic acid complex bound to the target molecule.

[A44] The method according to [A43], wherein the eluting in the step (3A) is performed by treating with an enzyme, heating, applying a chemical stimulus, or irradiating with a light.

[A45] The method according to [A44], wherein the enzyme is a TEV protease.

[A46] The method according to [A44], wherein the light to be irradiated is a light having a wavelength of 300 nm or more and 500 nm or less.

[A47] The method according to [A44], wherein the chemical stimulus is an acid or a base.

[A48] The method according to any of [A1] to [A47], further comprising, between the steps (3) and (4), a step (3B) of identifying an amino acid sequence of the peptide moiety of the barcoded peptide-nucleic acid complex bound to the target molecule.

[A49] The method according to [A48], wherein the amino acid sequence is identified based on a nucleic acid sequence of the nucleic acid moiety of the barcoded peptide-nucleic acid complex bound to the target molecule.

[A50] The method according to [A48] or [A49], wherein the amino acid sequence is identified based on a nucleic acid sequence of the nucleic acid moiety of the barcoded peptide-nucleic acid complex bound to the target molecule and a barcode sequence.

[A51] The method according to any of [A1] to [A50], wherein in the step (4), a nucleic acid corresponding to the nucleic acid moiety of the barcoded peptide-nucleic acid complex having the same barcode sequence among the barcoded peptide-nucleic acid complexes bound to the target molecule is selectively amplified.

[A52] The method according to any of [A1] to [A51], wherein the barcode primer is a second primer containing a barcode sequence.

[A53] The method according to any of [A1] to [A52], wherein the barcode primer is a second primer consisting of the barcode sequence.

[A54] The method according to [A53], wherein the barcode sequence has 15-30 bases.

[A55] The method according to any of [A1] to [A54], wherein in the step (4), a nucleic acid of the barcoded peptide-nucleic acid complex having the same barcode sequence as a barcode sequence of the barcode primer is selectively amplified.

[A56] The method according to any of [A1] to [A55], wherein in the step (4), the nucleic acid is amplified by a PCR method.

[A57] The method according to any of [A1] to [A56], wherein the amplified nucleic acid has the same base as DNA contained in the barcoded peptide-nucleic acid complex.

[A58] The method according to any of [A1] to [A57], wherein the steps (1) to (4) constitute one cycle, and the cycle is repeated multiple times.

[A59] The method according to any of [A1] to [A58], further comprising, after the step (4) or before starting a next cycle, a step (5) of further amplifying the nucleic acid amplified in the step (4) with a primer that does not contain a barcode sequence.

[A60] The method according to any of [A1] to [A58], comprising, after the step (4) or before starting a next cycle, a step (5) of further amplifying the nucleic acid amplified in the step (4) with a primer that does not contain a barcode sequence, wherein a nucleic acid that does not contain the barcode sequence is amplified.

[A61] The method according to any of [A1] to [A60], wherein the primer that does not contain a barcode sequence is a primer annealing to the 3' region of the nucleic acid library.

[A62] The method according to any of [A1] to [A61], wherein the nucleic acid display library is a library having a diversity of $10^4$ or more.

[A63] The method according to any of [A1] to [A60], wherein the nucleic acid display library is a library having a diversity of $10^{10}$ or more.

[A64] The method according to any of [A1] to [A63], wherein the nucleic acid display library is a library containing $10^4$ or more types of peptide-nucleic acid complexes.

[A65] The method according to any of [A1] to [A64], wherein the nucleic acid display library is a library containing $10^{10}$ or more types of peptide-nucleic acid complexes.

[A66] The method according to any of [A1] to [A65], wherein the nucleic acid display library is an mRNA display library.

[A67] The method according to any of [A1] to [A66], wherein the target molecule is a protein.
[B1] A method for screening for a candidate peptide capable of binding to a target molecule, the method comprising the steps of:

(1) independently translating a plurality of nucleic acid libraries using a cell-free translation system to prepare a peptide-nucleic acid complex containing a nucleic acid moiety and a peptide moiety,
(1-1) barcoding the peptide-nucleic acid complex to prepare a plurality of nucleic acid display libraries containing the barcoded peptide-nucleic acid complex;
(2) mixing the plurality of nucleic acid display libraries to prepare a mixed nucleic acid display library;
(3) bringing the mixed nucleic acid display library into contact with the target molecule; and
(4) amplifying a nucleic acid contained in the nucleic acid moiety of the barcoded peptide-nucleic acid complex bound to the target molecule, using a barcode primer.

[B2] The method according to [B1], wherein the barcoded peptide-nucleic acid complex contains a barcoded nucleic acid sequence (barcode sequence) in the nucleic acid moiety.
[B3] The method according to [B1] or [B2], comprising the characteristics described in any of [A2] to [A67].

The "characteristics described in any of [A2] to [A67]" means the matter specifying the invention described in each of the above [A2] to [A67], and the terms used in common with the descriptions in [B1] and [B2] are applied and understood interchangeably.
[C1] A method for screening for a candidate peptide capable of binding to a target molecule, the method comprising the steps of:

(1) independently translating a plurality of nucleic acid libraries using a single cell-free translation system to prepare a peptide-nucleic acid complex,
(1-1) barcoding the peptide-nucleic acid complex to prepare a plurality of nucleic acid display libraries containing the barcoded peptide-nucleic acid complex;
(2) mixing the plurality of nucleic acid display libraries to prepare a mixed nucleic acid display library;
(3) bringing the mixed nucleic acid display library into contact with the target molecule; and
(4) amplifying a nucleic acid contained in the nucleic acid moiety of the barcoded peptide-nucleic acid complex bound to the target molecule, using a barcode primer.

[C2] The method according to [C1], wherein a nucleic acid sequence of a nucleic acid contained in the nucleic acid library contains a random sequence, and the random sequence contains at least two triplets of one or more types selected from a plurality of types of triplets.
[C3] The method according to [C1] or [C2], wherein the nucleic acid library has a different number of repeats from other nucleic acid libraries.
[C4] The method according to any of [C1] to [C3], wherein the peptide moiety of the peptide-nucleic acid complex contains a predetermined amino acid, and the nucleic acid moiety contains a triplet encoding the amino acid.
[C5] The method according to any of [C1] to [C4], wherein the plurality of nucleic acid display libraries are nucleic acid display libraries, each of which is produced by translation based on the same genetic code table.
[C6] The method according to any of [C1] to [C5], comprising the characteristics described in any of [A1] to [A67].
[D1] A method for producing a nucleic acid display library, the method comprising steps (a) and (b) of:

(a) designing a nucleic acid display library containing a peptide-nucleic acid complex using an analysis result of principal component analysis with a descriptor as an index;
(b) translating a nucleic acid library using a cell-free translation system to prepare a peptide-nucleic acid complex.

[D2] The method according to [D1], further comprising, after the step (b), a step (c) of barcoding the peptide-nucleic acid complex to prepare the nucleic acid display library containing the barcoded peptide-nucleic acid complex.
[D3] The method according to [D1] or [D2], further comprising a step (d) of separately preparing a nucleic acid display library to provide a plurality of nucleic acid display libraries.
[D4] The method according to any of [D1] to [D3], wherein the plurality of nucleic acid display libraries is libraries with little structural overlap of the peptide-nucleic acid complexes among the plurality of nucleic acid display libraries.
[D5] The method according to any of [D1] to [D4], wherein the descriptor is descriptors including 'NumValenceElectrons', 'PEOE_VSA3', 'fr_A1_OH', 'fr_Al_OH_noTert', 'fr_Ar_N', 'fr_C_O_noCOO', 'fr_C_S', 'fr_HOCCN', 'fr_NH1',

'fr_Ndealkylation2', 'fr_Nhpyrrole', 'fr_SH', 'fr_alkyl_halide', 'fr_allylic_oxid', 'fr_aryl_methyl', 'fr_azide', 'fr_azo', 'fr_bicyclic', 'fr_diazo', 'fr_dihydropyridine', 'fr_epoxide', 'fr_ether', 'fr_furan', 'fr_halogen', 'fr_hdrzine', 'fr_hdrzone', 'fr_imidazole', 'fr_imide', 'fr_isocyan', 'fr_isothiocyan', 'fr_ketone', 'fr_ketone_Topliss', 'fr_lactam', 'fr_lactone', 'fr_methoxy', 'fr_morpholine', 'fr_nitrile', 'fr_nitro', 'fr_nitro_arom', 'fr_nitro_arom_nonortho', 'fr_nitroso', 'fr_oxazole', 'fr_phenol', 'fr_phenol_noOrthoHbond, 'fr_phos_acid', 'fr_phos_ester', 'fr_piperdine', 'fr_piperzine', 'fr_priamide', 'fr_prisulfonamd', 'fr_pyridine', 'fr_quatN', 'fr_sulfide', 'fr_sulfonamd', 'fr_sulfone', 'fr_term_acetylene', 'fr_tetrazole', 'fr_thiazole', 'fr_thiocyan', and 'fr_thiophene'.

[D6] The method according to any of [D1] to [D5], wherein the nucleic acid display library is a library having a diversity of $10^4$ or more.

[D7] The method according to any of [D1] to [D5], wherein the nucleic acid display library is a library containing $10^4$ or more types of peptide-nucleic acid complexes.

[D8] The method according to any of [D1] to [D7], wherein the nucleic acid display library is an mRNA display library.

[D9] A nucleic acid display library obtained by the method according to any of [D1] to [D8].

[D10] The method according to any of [A1] to [A67], wherein the nucleic acid display library is the library according to [D9].

The "characteristics described in any of [A1] to [A67]" means the matter specifying the invention described in each of the above [A1] to [A67], and the terms used in common with the descriptions in [C1] to [C4] are applied and understood interchangeably. In the above numberings, the number cited in the dependent item includes the branch number of the number, unless otherwise mentioned. For example, the [A1] cited in the dependent item shows that not only [A1] but also its branch number such as [A1.1] are included. The same applies to other numberings.

[Advantageous Effects of Invention]

The screening method according to the present invention enables simultaneous screening of a plurality of libraries and more efficient finding of peptides that bind to a target molecule. In addition, the screening method according to the present invention can not only expand the library size by using a mixed nucleic acid display library composed of a plurality of mixed libraries, but also easily identify which library each peptide is derived from, based on the barcode sequence bound to each peptide. Furthermore, by selectively amplifying a nucleic acid moiety encoding the peptide using a primer corresponding to each barcode sequence, a peptide capable of binding to the target molecule can be enriched according to each library, so that it is difficult to overlook the peptide with low enrichment, and the peptide capable of binding to the target molecule can be efficiently found.

[Brief Description of Drawings]

[Figure 1] Figure 1 is a plot of the values of each principal component of the random sequences in each codon table (Random1: PURE1, Random2: PURE2, Random3: PURE3, Random4: PURE4).

[Figure 2] Figure 2 is a plot of the values of each principal component of the enrichment sequence in each codon table.

[Figure 3] Figure 3 is a plot of the values of each principal component of enrichment sequences (shown in black) and all random sequences (shown in gray, where each shape corresponds to the following cell-free translation system: ○: PURE1, △: PURE2, □: PURE3, ×: PURE4).

[Description of Embodiments]

Hereinafter, one embodiment of the present invention is described in detail.

As used herein, the "amino acid" and "amino acid analog" constituting a peptide may be referred to as "amino acid residue" and "amino acid analog residue", respectively.

The "amino acid" as used herein is $\alpha$-, $\beta$-, or $\gamma$-amino acid, and is not limited to a naturally occurring amino acid and may be a non-naturally occurring amino acid. The naturally occurring amino acid in the present application refers to 20 amino acids contained in a protein, specifically, Gly, Ala, Ser, Thr, Val, Leu, Ile, Phe, Tyr, Trp, His, Glu, Asp, Gln, Asn, Cys, Met, Lys, Arg, and Pro. In the case of $\alpha$-amino acids, they may be either L-amino acids or D-amino acids, or $\alpha,\alpha$-dialkylamino acids. A side chain of the amino acid is not particularly limited, and the side chain is freely selected from, in addition to a hydrogen atom, an alkyl group, an alkenyl group, an alkynyl group, an aryl group, a heteroaryl group, an aralkyl group, a cycloalkyl group, and the like. A substituent may be added to each of the side chain, and these substituents are freely selected, for example, from any functional groups including an N atom, an O atom, an S atom, a B atom, a Si atom, and a P atom (i.e., an optionally substituted alkyl group, alkenyl group, alkynyl group, aryl group, heteroaryl group, aralkyl

group, cycloalkyl group, and the like).

Examples of the substituent include halogen-derived substituents such as fluoro (-F), chloro (-Cl), bromo (-Br), and iodo (-I). Further examples include those substituted with one or more of these, such as an alkyl group, an cycloalkyl group, an alkenyl group, an alkynyl group, an aryl group, a heteroaryl group, or an aralkyl group which may have halogen as a substituent.

Examples of the substituent for forming an ether as a substituent derived from an O atom include an alkoxy group (-OR), and the alkoxy group is selected from an alkylalkoxy group, a cycloalkylalkoxy group, an alkenylalkoxy group, an alkynylalkoxy group, an arylalkoxy group, a heteroarylalkoxy group, an aralkylalkoxy group, and the like. Examples of the substituent for forming an alcohol moiety include a hydroxyl group (-OH). Examples of the substituent for forming a carbonyl group include a carbonyl group (-C(=O)-R), and the carbonyl group is selected from a hydrocarbonyl group (-C=O-H, aldehyde is obtained as a compound), an alkylcarbonyl group (a ketone is obtained as a compound), a cycloalkylcarbonyl group, an alkenylcarbonyl group, an alkynylcarbonyl group, an arylcarbonyl group, a heteroarylcarbonyl group, an aralkylcarbonyl group, and the like. Examples of the substituent for forming a carboxylic acid ($-CO_2H$) include a carboxyl group. Examples of the substituent for forming an ester group include an oxycarbonyl group (-O-C(=O)-R) and a carbonylalkoxy group (-C(=O)-OR). The carbonylalkoxy group is selected from an alkyloxycarbonyl group, a cycloalkyloxycarbonyl group, an alkenyloxycarbonyl group, an alkynyloxycarbonyl group, an aryloxycarbonyl group, a heteroaryloxycarbonyl group, an aralkyloxycarbonyl groups, and the like, and the oxycarbonyl group is selected from an alkylcarbonyloxy group, a cycloalkylcarbonyloxy group, an alkenylcarbonyloxy group, an alkynylcarbonyloxy group, an arylcarbonyloxy group, a heteroarylcarbonyloxy group, an aralkylcarbonyloxy group, and the like.

Examples of the substituent for forming a thioester include a mercaptocarbonyl group (-S-C(=O)-R) and a carbonylalkylmercapto group (-C(=O)-SR), which are selected from a mercaptoalkylcarbonyl group, a mercaptocycloalkylcarbonyl group, a mercaptoalkenylcarbonyl group, a mercaptoalkynylcarbonyl group, a mercaptoarylcarbonyl group, a mercaptoheteroarylcarbonyl group, a mercaptoaralkylcarbonyl group, and the like, or a carbonylalkylmercapto group, a carbonylcycloalkylmercapto group, a carbonylalkenylmercapto group, a carbonylalkynylmercapto group, a carbonylarylmercapto group, a carbonylheteroarylmercapto group, a carbonylaralkylmercapto group, and the like.

Examples of the substituent for forming an amide group include an aminoalkylcarbonyl group (-NH-C(=O)-R), an aminocycloalkylcarbonyl group, an aminoalkenylcarbonyl group, an aminoalkynylcarbonyl group, an aminocycloalkylcarbonyl group, an aminoarylcarbonyl group, an aminoheteroarylcarbonyl group, an aminoaralkylcarbonyl group, and the like or a carbonylalkylamino group (-C(=O)-NHR), a carbonylcycloalkylamino group, a carbonylalkenylamino group, a carbonylalkynylamino group, a carbonylarylamino group, a carbonylheteroarylamino group, a carbonylaralkylamino group, and the like. Further examples include a compound in which an H atom bound to the N atom is replaced with an alkyl group, a cycloalkyl group, an alkenyl group, an alkynyl group, an aryl group, a heteroaryl group, or an aralkyl group.

Examples of the substituent for forming a carbamate group include an aminoalkylcarbamate group (-NH-C(=O)-OR), an aminocycloalkylcarbamate group, an aminoalkenylcarbamate group, an aminoalkynylcarbamate group, an aminocycloalkylcarbamate group, an aminoarylcarbamate group, an aminoheteroarylcarbamate group, and an aminoaralkylcarbamate group. Further examples include a compound in which an H atom bound to the N atom is replaced with an alkyl group, a cycloalkyl group, an alkenyl group, an alkynyl group, an aryl group, a heteroaryl group, or an aralkyl group.

Examples of the substituent for forming a sulfonamide group include an aminoalkylsulfonyl group ($-NH-SO_2-R$), an aminocycloalkylsulfonyl group, an aminoalkenylsulfonyl group, an aminoalkynylsulfonyl group, an aminocycloalkylsulfonyl group, an aminoarylsulfonyl group, an aminoheteroarylsulfonyl group, an aminoaralkylsulfonyl group, and the like or a sulfonylalkylamino group ($-SO_2-NHR$), a sulfonylcycloalkylamino group, a sulfonylalkenylamino group, a sulfonylalkynylamino group, a sulfonylarylamino group, a sulfonylheteroarylamino group, a sulfonylaralkylamino group, and the like. Further examples include a compound in which an H atom bound to the N atom is replaced with an alkyl group, a cycloalkyl group, an alkenyl group, an alkynyl group, an aryl group, a heteroaryl group, or an aralkyl group.

Examples of the substituent for forming a sulfamide group include an aminoalkylsulfamoyl group ($-NH-SO_2-NHR$), an aminocycloalkylsulfamoyl group, an aminoalkenylsulfamoyl group, an aminoalkynylsulfamoyl group, an aminocycloalkylsulfamoyl group, an aminoarylsulfamoyl group, an aminoheteroarylsulfamoyl group, and an aminoaralkylsulfamoyl group. Further examples include a compound in which H atoms bound to the N atoms are replaced with substituents selected from an alkyl group, a cycloalkyl group, an alkenyl group, an alkynyl group, an aryl group, a heteroaryl group, and an aralkyl group, which may consist of any two identical or different substituents, or may form a cyclic structure.

Examples of the substituent for forming a thiocarboxylic acid include a thiocarboxylic acid group (-C(=O)-SH), and examples of a functional group for forming a keto acid include a keto acid group ($-C(=O)-CO_2H$).

Examples of the substituent for forming a thiol group as a substituent derived from an S atom include a thiol group (-SH), and an alkylthiol, a cycloalkylthiol, an alkenylthiol, an alkynylthiol, an arylthiol, a heteroarylthiol, and an aralkylthiol are formed. A substituent for forming thioether (-S-R) is selected from an alkylmercapto group, a cycloalkylmercapto group, an alkenylmercapto group, an alkynylmercapto group, an arylmercapto group, a heteroarylmercapto group, an aralkylmercapto group, and the like. A substituent for forming a sulfoxide group (-S(=O)-R) is selected from an

alkylsulfoxide group, a cycloalkylsulfoxide group, an alkenylsulfoxide group, an alkynylsulfoxide group, an arylsulfoxide group, a heteroarylsulfoxide group, an aralkylsulfoxide group, and the like. A substituent for forming a sulfone group ($-SO_2-R$) is selected from an alkylsulfone group, a cycloalkylsulfone group, an alkenylsulfone group, an alkynylsulfone group, an arylsulfone group, a heteroarylsulfone group, an aralkylsulfone group, and the like. Examples of the substituent for forming a sulfonic acid include a sulfonic acid group ($-SO_3H$).

Examples of the substituent derived from an N atom include an azide group ($-N_3$); a nitrile group (-CN); an amino group ($-NH_2$) as a substituent for forming a primary amine; an alkylamino group, a cycloalkylamino group, an alkenylamino group, an alkynylamino group, an arylamino group, a heteroarylamino group, an aralkylamino group, and the like as a substituent for forming a secondary amine (-NH-R); substituents selected from an alkyl group, a cycloalkyl group, an alkenyl group, an alkynyl group, an aryl group, a heteroaryl group, an aralkyl group, and the like, which may consist of any two identical or different substituents, or may form a cyclic structure, as substituent for forming a tertiary amine (-NR(R')), such as an alkyl(aralkyl)amino group; an amidino group ($-C(=NH)-NH_2$), or one in which three substituents on the N atom are substituted with any three identical or different groups of an alkyl group, a cycloalkyl group, an alkenyl group, an alkynyl group, an aryl group, a heteroaryl group, and an aralkyl group as substituents for forming an amidino group (-C(=NR)-NR'R"), such as an alkyl(aralkyl)(aryl)amidino group; and a guanidine group ($-NH-C(=NH)-NH_2$) or one in which R, R', R", R‴ are selected from an alkyl group, a cycloalkyl group, an alkenyl group, an alkynyl group, an aryl group, a heteroaryl group, an aralkyl group, which may consist of any four identical or different substituents or may form a cyclic structure, as substituents for forming a guanidino group (-NR-C(=NR")-NR'R").

Examples of the substituent for forming a urea group include an aminocarbamoyl group (-NR-C(=O)-NR'R"). Examples of R, R' and R" include substituents selected from a hydrogen atom, an alkyl group, a cycloalkyl group, an alkenyl group, an alkynyl group, an aryl group, a heteroaryl group, and an aralkyl group, which may consist of any three identical or different substituents, or may form a cyclic structure.

Examples of a functional group derived from a B atom include alkylborane (-BR(R')) and alkoxyborane (-B(OR)(OR')). Examples of the two substituents include substituents selected from an alkyl group, a cycloalkyl group, an alkenyl group, an alkynyl group, an aryl group, a heteroaryl group, and an aralkyl group, which may consist of any two identical or different substituents, or may form a cyclic structure.

In this way, one or two or more functional groups containing an O atom, an N atom, an S atom, a B atom, a P atom, a Si atom, and a halogen atom usually used in a low molecular weight compound, such as a halogen group, may be added. That is, one or more further substituents may be added to the alkyl group, the cycloalkyl group, the alkenyl group, the alkynyl group, the aryl group, the heteroaryl group, or the aralkyl group shown as one of the substituents. The conditions for functional groups satisfying all of these are defined as free selection of substituents. Any conformation is acceptable for β- and γ-amino acids as in α-amino acids, and the selection of the side chain is also the same as in α-amino acids without particular limitation.

The backbone amino group moiety of the amino acid may be unsubstituted ($NH_2$ group) or substituted (that is, an NHR group: R represents an optionally substituted alkyl group, alkenyl group, alkynyl group, aryl group, heteroaryl group, aralkyl group, or cycloalkyl group. Alternatively, the carbon atom bound to the N atom and the carbon atom on the side chain extending from the carbonyl α position may be combined to form a ring, such as proline. The substituent can be freely selected, and examples thereof include a halogen group, an ether group, and a hydroxy group). Such an amino acid having the substituted backbone amino group is referred to herein as the "N-substituted amino acid". The "N-substituted amino acid" as used herein is preferably an N-alkyl amino acid, and may be N-C1-C6 alkyl amino acids or N-$C_1$-$C_4$ alkyl amino acid. More preferably, N-substituted amino acid may be an N-methylamino acid or an N-ethyl amino acid, particularly preferably an N-methylamino acid.

The "amino acid analog" as used herein means an α-hydroxycarboxylic acid. The α-hydroxycarboxylic acid may have a variety of substituents as a side chain at the α position of the hydroxy group, as in amino acids. When the α-hydroxycarboxylic acid has the above side chain, the carbon atom to which the side chain is attached can be an asymmetric center. The three-dimensional structure of the α-hydroxycarboxylic acid may correspond to the L-type or the D-type of the amino acid. The selection of the side chain is not particularly limited, and is freely selected from, for example, an alkyl group, an alkenyl group, an alkynyl group, an aryl group, a heteroaryl group, an aralkyl group, a cycloalkyl group, and the like, which are optionally substituted. The number of substituents is not limited to one and may be two or more. For example, a substituent has an S atom, and may further have a functional group such as an amino group or a halogen group.

The "amino acid" and "amino acid analog" constituting a peptide include all combinations of isotopes corresponding to each atom. The isotope of the "amino acid" and "amino acid analog" is a form in which at least one atom is replaced with an atom having the same atomic number (proton number) and a different mass number (total number of protons and neutrons). Examples of the atom contained in the "amino acid" or "amino acid analog" constituting a peptide include a hydrogen atom, a carbon atom, a nitrogen atom, an oxygen atom, a phosphorus atom, a sulfur atom, a fluorine atom, a chlorine atom, and the like, and the isotopes thereof include $^{2}H$, $^{3}H$; $^{13}C$, $^{14}C$; $^{15}N$; $^{17}O$, $^{18}O$; $^{31}P$, $^{32}P$; $^{35}S$; $^{18}F$; $^{36}Cl$; and the like, respectively.

The "peptide" as used herein is not limited as long as two or more natural and/or non-natural amino acids are

linked by an amide bond, and it may have an ester bond in a part of the backbone, such as a depsipeptide. The number of amino acid residues contained in the peptide is preferably 5 to 30 residues, more preferably 7 to 20 residues. The peptide may be a linear peptide, a cyclic peptide, or a peptide of a structure in which these are attached.

The "nucleic acid" as used herein includes a deoxyribonucleic acid (DNA), a ribonucleic acid (RNA), a nucleotide derivative having an artificial base, and a peptide nucleic acid (PNA), and may be any mixture thereof as long as the genetic information of interest is retained. That is, DNA-RNA hybrid nucleotides or chimeric nucleic acids in which different nucleic acids such as DNA and RNA are linked in a single strand are also included in the nucleic acids in the present invention.

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule; and
(4) amplifying a nucleic acid corresponding to the nucleic acid moiety of the barcoded peptide-nucleic acid complex bound to the target molecule, using a barcode primer.

<u>Step (1)</u>

The step (1) is a step of preparing a plurality of nucleic acid display libraries containing a barcoded peptide-nucleic acid complex.

The "nucleic acid display library" as used herein means a library in which a peptide is associated with an RNA or DNA encoding the peptide. A peptide that binds to a desired target can be enriched by bringing a library into contact with the desired target and washing away molecules that are not bound to the target (panning method). It is possible to identify the sequence of the protein bound to the target by analyzing the genetic information associated with the peptide selected through such a process. For example, a method of utilizing the nonspecific linkage of an antibiotic puromycin, an analogue of aminoacyl tRNA, to a protein in mRNA translation extension by ribosomes has been reported as mRNA display (Proc Natl Acad Sci USA. 1997; 94: 12297-302. RNA-peptide fusions for the in vitro selection of peptides and proteins. Roberts RW, Szostak JW.) or in vitro virus (FEBS Lett. 1997;414:405-8. In vitro virus: bonding of mRNA bearing puromycin at the 3'-terminal end to the C-terminal end of its encoded protein on the ribosome in vitro. Nemoto N, Miyamoto-Sato E, Husimi Y, Yanagawa H.).

The peptide-nucleic acid complex contains a nucleic acid moiety and a peptide moiety, wherein the nucleic acid moiety contains a nucleic acid sequence of a nucleic acid encoding a peptide contained in the peptide moiety. The peptide moiety of the peptide-nucleic acid complex contains a predetermined amino acid, and the nucleic acid moiety contains a triplet encoding the amino acid.

The "triplet" as used herein means a permutation of three consecutive nucleobases in a nucleic acid sequence, usually written in order from the 5' end. When the nucleic acid is DNA, examples of the triplet include TTT, TTG, CTT, CTG, ATT, ATG, GTT, GTG, TCT, TCG, CCG, ACT, GCT, TAC, CAT, CAG, AAC, GAT, GAG, TGC, TGG, CGT, CGG, AGT, AGG, and GGT. These correspond to codons. When translated using a cell-free translation system, the same triplet is translated into the same amino acid, so that a peptide produced by translation of an mRNA containing a plurality of identical triplets will contain a plurality of identical amino acids.

The nucleic acid sequence of a nucleic acid encoding a peptide contained in the peptide moiety contains a random sequence. The random sequence herein is a sequence that is different (sequence that is not common) for each nucleic acid sequence and a sequence consisting of arbitrarily selected amino acids and/or amino acid analogs. The random sequence may contain at least one triplet (codon) specifying an unnatural amino acid. The random sequence preferably contains at least one, 2 to 1000, 2 to 750, 2 to 500, 2 to 250, 2 to 100, 2 to 50, 2 to 20, 2 to 18, 2 to 15, 2 to 12, at least 3, 3 to 1000, 3 to 750, 3 to 500, 3 to 250, 3 to 100, 3 to 50, 3 to 18, 3 to 15, 3 to 12, at least 4, 4 to 1000, 4 to 750, 4 to 500, 4 to 250, 4 to 100, 4 to 50, 4 to 18, 4 It is preferable to include 15, 4 to 12, at least 5, 5 to 1000, 5 to 750, 5 to 500, 5 to 250, 5 to 100, 5 to 50, 5 to 18, 5 to 15, or 5 to 12 triplets selected from a plurality of types of triplets. As used herein, the number of triplets is referred to as "number of repeats". The number of types of triplets may be 64 or less, 60 or less, 55 or less, 50 or less, 45 or less, 40 or less, 35 or less, 30 or less, 25 or less, 20 or less, 15 or less, 10 or less, or 5 or less, and preferably 64 or less, more preferably 50 or less, and particularly preferably 40 or less. Each triplet may be the same or different from each other, and may be randomly selected. It is preferable that the peptide moiety of the peptide-nucleic acid complex contains a predetermined amino acid, and the nucleic acid moiety contains a triplet encoding the amino acid.

The peptide-nucleic acid complex may be bound to the nucleic acid at the C-terminal side of the peptide moiety. A

linker may be present between the nucleic acid moiety and the peptide moiety of the peptide-nucleic acid complex. The peptide-nucleic acid complex preferably contains a linker formed of puromycin or a derivative thereof, a polymer of RNA, DNA, or hexaethylene glycol (spc18: 18-O-Dimethoxytritylhexaethyleneglycol, 1-[(2-cyanoethyl)-(N,N-diisopropyl)]-phosphoramidite) or the like as a linker. Specifically, for example, the 3' end of the nucleic acid moiety and the C-terminal of the peptide moiety are linked via a linker containing a puromycin or the like. The peptide-nucleic acid complex is preferably a peptide-DNA complex or a peptide-mRNA complex, more preferably a peptide-mRNA complex, and particularly preferably a cyclic peptide-mRNA complex. A schematic diagram showing an example of the peptide-nucleic acid complex is shown below.

[Formula 1]

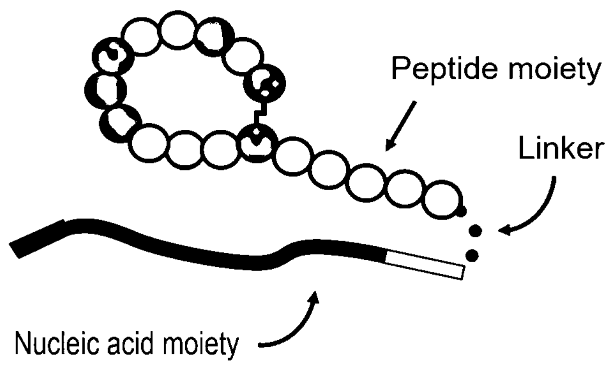


When a cell-free translation system is used, it is preferable to include a nucleic acid sequence encoding a spacer (a linear moiety contained in the peptide moiety and linked to a nucleic acid) downstream of the nucleic acid of interest. Examples of the spacer sequence include, but are not limited to, a sequence containing glycine or serine.

The peptide-nucleic acid complex can be produced, for example, by the method described in WO 2013/100132. Specifically, the peptide-nucleic acid complex can be produced by a method including the steps of:

A) acylating dinucleotide pdCpA or pCpA with a desired amino acid or amino acid analog to provide aminoacyl pdCpA or aminoacyl pCpA;
B) providing a 3'-terminal CA-deficient tRNA;
C) linking the aminoacyl pdCpA or aminoacyl pCpA of the step A) to the CA-deficient tRNA of the step B) to provide an initiator tRNA acylated with a desired amino acid or amino acid analog,
D) providing a cell-free translation system containing the initiator tRNA of the step C) and free of at least one of methionine, a methionyl tRNA synthase (MetRS), a translation initiator tRNA for methionine, a formyl donor, and a methionyl tRNA transferase;
E) providing a template DNA in which a cysteine codon UGU or UGC follows a translation initiation ATG downstream of a promoter, and a peptide sequence containing a codon corresponding to an anticodon of the tRNA of the step C) is located further downstream,
F) providing mRNA from the template DNA of the step E),
G) linking a linker to the 3' end of the mRNA of the step F), and
H) adding the mRNA bound to the linker of the step G) to the cell-free translation system of the step D) and translating to provide a pre-cyclization peptide-mRNA complex.

When the peptide is a cyclic peptide, a step of further cyclizing the pre-cyclization peptide-mRNA complex of the step H) may be provided. In the step H), a desulfurization reaction can be carried out as needed. In addition, after the step G), a step of synthesizing cDNA using a primer annealing to the 3' region of the mRNA can be included.

In the steps A to C, RNA can be synthesized by preparing a template DNA that encodes a desired tRNA sequence and disposes a T7, T3 or SP6 promoter upstream and transcribing with RNA polymerases adapted for promoters, such as T7 RNA polymerase and T3, and SP6 RNA polymerase. A generated tRNA of interest can also be extracted by extracting and purifying tRNA from the cells and using probes of complementary sequences of tRNA sequence. At this time, the cells transformed with an expression vector of tRNA of interest can be used as a source. RNA of the sequence of interest can also be synthesized by chemical synthesis. For example, an aminoacyl tRNA can be obtained by binding tRNA in which CA is removed from the CCA sequence at the 3' end obtained in this way to an aminoacylated pdCpA prepared separately with an RNA ligase (pdCpA method). Alternatively, full-length tRNA is prepared and aminoacylation by flexizyme, which is a ribozyme that makes active esters of various unnatural amino acids carried on tRNA, can be performed. Acylated tRNA

can also be obtained by using the method described below.

In the steps E to H, a DNA in which a desired nucleotide sequence is placed downstream of a promoter, such as a T7 promoter, is first chemically synthesized, then used as a template to obtain a double-stranded DNA by a primer extension reaction. The obtained double-stranded DNA is transcribed as a template into mRNA using an RNA polymerase such as T7 RNA polymerase. A linker containing puromycin or the like is attached to the 3' end of the mRNA obtained by the transcription and the mRNA is translated into a protein using a known cell-free translation system such as PURE system, then puromycin is incorporated into the protein, and the mRNA and its encoded protein are linked via puromycin. In this way, a peptide-mRNA complex can be constructed in which the mRNA and its encoded product (peptide) are associated.

A transfer RNA (tRNA) is an RNA consisting of 73-93 bases of chain length containing a 3'-terminal CCA sequence with a molecular weight of 25,000 to 30,000, which forms a triplex with a polypeptide elongation factor (EF-Tu) and GTP as an aminoacylated tRNA esterically linked to the carboxy terminus of an amino acid via the 3'-terminus, is transported to a ribosome, and is involved in the identification of a codon by the formation of a base pair between the anticodon in the sequence of the tRNA and the codon of the mRNA in the translation of the nucleic acid sequence information of the mRNA into an amino acid sequence by the ribosome. Intracellularly biosynthesized tRNAs contain a covalently modified base, which may influence the conformation of the tRNA and the base pair formation of the anticodon, and may aid in the codon identification by the tRNA. While tRNAs synthesized by general in vitro transcription consist of the so-called nucleobases adenine, uracil, guanine, and cytosine, intracellularly prepared or chemically synthesized tRNAs may contain modified bases such as methylated bases, sulfur-containing derivatives, deamino derivatives, and adenosine derivatives containing isopentenyl groups and threonine, and may also contain deoxybases when a pdCpA method or the like is used.

According to the general genetic code table (also called "codon table"), methionine is translated as a translation initiation amino acid. However, a method of translating the N-terminus as a desired amino acid can be used by translating with an acyl tRNA to which the desired amino acid or amino acid analog is attached. It is known that the N-terminal introduction of unnatural amino acids has a higher allowance for amino acids than extension, and amino acids or amino acid analogs with a structure greatly different from that of natural amino acids can be used (see Non Patent Literature: J Am Chem Soc. 2009 Apr 15;131(14):5040-1. Translation initiation with initiator tRNA charged with exotic peptides. Goto Y, Suga H.). For example, in the present invention, methods for introducing a desired amino acid or amino acid analog other than methionine to the N-terminus include the following method. A tRNA acylated with a desired amino acid or amino acid analog is added as the translation initiation tRNA to a translation system without methionine, formyl donor or methionyl transferase, and a translation initiation codon (e.g., ATG) and made to encode the amino acid or amino acid analog, and translation is performed to construct a pre-cyclization peptide with the amino acid or amino acid analog at the end. When various combinations of a translation initiation tRNA in which anticodon is not CAU and a codon corresponding to the anticodon are utilized as the combination of the translation initiation tRNA and an initiator codon, the N-terminus can be diversified. In other words, it is possible to create peptides or a library of peptides before cyclization in which the N-terminal residues are not limited to one type by aminoacylating desired amino acids, amino acid analogs or N-terminal carboxylic acid analogs to a plurality of types of translation initiation tRNAs of different anticodons, respectively, and translating mRNAs or a library of mRNAs with codons corresponding to those as the initiation codons. Specifically, a peptide or peptide library before cyclization can be created using, for example, the method described in Mayer C, et al. Anticodon sequence mutants of Escherichia coli initiator tRNA: effects of overproduction of aminoacyl-tRNA synthetases, methionyl-tRNA formyltransferase, and initiation factor 2 on activity in initiation. Biochemistry. 2003, 42, 4787-99. (translation initiation from an amino acid other than f-Met by E. coli with a mutation in an initiation tRNA having an anticodon other than CAU and expression of a protein containing the same codon in the middle).

A peptide can be translated by adding mRNA to PURE system mixed with protein factors required for the translation of E. coli (methionyl tRNA transformylase, EF-G, RF1, RF2, RF3, RRF, IF1, IF2, IF3, EF-Tu, EF-Ts, ARS (selected from AlaRS, ArgRS, AsnRS, AspRS, CysRS, GlnRS, GluRS, GlyRS, HisRS, IleRS, LeuRS, LysRS, MetRS, PheRS, ProRS, SerRS, ThrRS, TrpRS, TyrRS, ValRS as needed), ribosome, amino acid, creatine kinase, myokinase, inorganic pyrophosphatase, nucleoside diphosphate kinase, E. coli-derived tRNA, creatine phosphate, potassium glutamate, HEPES-KOH pH7.6, magnesium acetate, spermidine, dithiothreitol, GTP, ATP, CTP, UTP, or the like. When T7 RNA polymerase is added, transcription and translation from a template DNA containing a T7 promoter can also be performed in conjugation. At this time, a peptide containing an unnatural amino acid group can be translationally synthesized by adding a group of desired aminoacyl tRNAs or a group of unnatural amino acids (e.g., F-Tyr) that are allowed by ARS to the system (Kawakami T, et al. Ribosomal synthesis of polypeptoids and peptoid-peptide hybrids. J Am Chem Soc. 2008, 130, 16861-3., Kawakami T, et al. Diverse backbone-cyclized peptides via codon reprogramming. Nat Chem Biol. 2009, 5, 888-90.). Alternatively, ribosomes and mutants of EF-Tu or the like can be used to increase the efficiency of introduction of the unnatural amino acid by translation (Dedkova LM, et al. Construction of modified ribosomes for incorporation of D-amino acids into proteins. Biochemistry 2006, 45, 15541-51., Doi Y, et al. Elongation factor Tu mutants expand amino acid tolerance of protein biosynthesis system. J Am Chem Soc. 2007, 129, 14458-62., Park HS, et al. Expanding the genetic code of Escherichia coli with phosphoserine. Science 2011, 333, 1151-4.).

The cell-free translation system is a mixture of tRNA, amino acids, ATP, and the like with factors involved in translation extracted from the cells of the material. The cell-free translation system does not contain living cells. Examples of the cells of the material include E. coli (Methods Enzymol. 1983;101:674-90. Prokaryotic coupled transcription-translation. Chen HZ, Zubay G.), yeasts (J. Biol. Chem. 1979 254: 3965-3969. The preparation and characterization of a cell-free system from Saccharomyces cerevisiae that translates natural messenger ribonucleic acid. E Gasior, F Herrera, I Sadnik, C S McLaughlin, and K Moldave), wheat germ (Methods Enzymol. 1983;96:38-50. Cell-free translation of messenger RNA in a wheat germ system. Erickson AH, Blobel G.), rabbit reticulocytes (Methods Enzymol. 1983;96:50-74. Preparation and use of nuclease-treated rabbit reticulocyte lysates for the translation of eukaryotic messenger RNA. Jackson RJ, Hunt T.), HeLa cells (Methods Enzymol. 1996;275:35-57.Assays for poliovirus polymerase, 3D(Pol), and authentic RNA replication in HeLa S10 extracts. Barton DJ, Morasco BJ, Flanegan JB.), and insect cells (Comp Biochem Physiol B. 1989;93:803-6. Cell-free translation in lysates from Spodoptera frugiperda (Lepidoptera:Noctuidae) cells. Swerdel MR, Fallon AM.). RNA polymerase, such as T7 RNA polymerase, can be added to the cells of the material to conjugate transcription and translation from DNA. Meanwhile, PURE system is a reconstituted cell-free translation system in which protein factors, energy regeneration system enzymes, and ribosomes necessary for translation in E. coli are extracted and purified, and mixed with tRNA, amino acids, ATP, GTP, and the like. PURE system not only has a low impurity content, but also allows easy preparation of systems that do not contain protein factors or amino acids that are desired to be eliminated, because it is a reconstitution system ((i) Nat Biotechnol. 2001; 19: 751-5. Cell-free translation reconstituted with purified components. Shimizu Y, Inoue A, Tomari Y, Suzuki T, Yokogawa T, Nishikawa K, Ueda T. (ii) Methods Mol Biol. 2010; 607: 11-21. PURE technology. Shimizu Y, Ueda T.). The reconstituted cell-free translation system can be created using an appropriate known method.

Various factors contained in the cell-free translation system, such as ribosomes and tRNA, can be purified from E. coli cells and yeast cells by methods well known to those skilled in the art. As the tRNA and aminoacyl tRNA synthetase (also referred to herein as "ARS"), not only naturally occurring ones, but also artificial tRNAs and artificial aminoacyl tRNA synthetases that recognize an unnatural amino acid can be used. With the artificial tRNA and artificial aminoacyl tRNA synthetase, it is possible to synthesize a peptide in which an unnatural amino acid is site-specifically introduced.

In introducing an unnatural amino acid into a peptide by translation, aminoacylation of tRNA which attains orthogonality and efficient incorporation into ribosomes ((i) Biochemistry 2003; 42: 9598-608. Adaptation of an orthogonal archaeal leucyl-tRNA and synthetase pair for four-base, amber, and opal suppression. Anderson JC, Schultz PG., (ii) Chem Biol. 2003; 10: 1077-84. Using a solid-phase ribozyme aminoacylation system to reprogram the genetic code. Murakami H, Kourouklis D, Suga H.) is required. Examples of the method for aminoacylating tRNA can include the following methods.

Inside cells, aminoacyl tRNA synthase that acylates tRNA with an amino acids is provided for each amino acid independently. Thus, a method of using certain aminoacyl tRNA synthase that allows unnatural amino acids such as N-Me-His, and a method of preparing and utilizing a modified aminoacyl tRNA synthetase that allows unnatural amino acids can be used ((i) Proc. Natl. Acad. Sci. U S A. 2002;99:9715-20. An engineered Escherichia coli tyrosyl-tRNA synthetase for site-specific incorporation of an unnatural amino acid into proteins in eukaryotic translation and its application in a wheat germ cell-free system. Kiga D, Sakamoto K, Kodama K, Kigawa T, Matsuda T, Yabuki T, Shirouzu M, Harada Y, Nakayama H, Takio K, Hasegawa Y, Endo Y, Hirao I, Yokoyama S.; (ii) Science 2003;301:964-7.An expanded eukaryotic genetic code.Chin JW, Cropp TA, Anderson JC, Mukherji M, Zhang Z, Schultz PG. Chin,JW.; (iii) Proc. Natl. Acad. Sci. U S A. 2006;103:4356-61.Enzymatic aminoacylation of tRNA with unnatural amino acids. Hartman MC, Josephson K, Szostak JW.). A method of aminoacylating tRNA and then chemically modifying an amino acid in a test tube can also be used (J Am Chem Soc. 2008; 130: 6131-6. Ribosomal synthesis of N-methyl peptides. Subtelny AO, Hartman MC, Szostak JW.). An aminoacyl tRNA can also be obtained by binding tRNA in which CA is removed from the CCA sequence at the 3' end to an aminoacylated pdCpA prepared separately with an RNA ligase (Biochemistry 1984; 23: 1468-73. T4 RNA ligase mediated preparation of novel "chemically misacylated" tRNAPheS. Heckler TG, Chang LH, Zama Y, Naka T, Chorghade MS, Hecht SM.). Aminoacylation by flexizyme, which is a ribozyme that makes active esters of various unnatural amino acids carried on tRNA, can also be used (J Am Chem Soc. 2002; 124: 6834-5. Aminoacyl-tRNA synthesis by a resin-immobilized ribozyme. Murakami H, Bonzagni NJ, Suga H.). A method of ultrasonically mixing tRNA and amino acid active esters in a cationic micelle can also be used (Chem Commun (Camb). 2005; (34): 4321-3. Simple and quick chemical aminoacylation of tRNA in cationic micellar solution under ultrasonic agitation. Hashimoto N, Ninomiya K, Endo T, Sisido M.). Amino acid acylation is also possible by adding an amino acid active ester bound to PNA complementary to near the 3' end of tRNA to tRNA (J Am Chem Soc. 2004; 126: 15984-9. In situ chemical aminoacylation with amino acid thioesters linked to a peptide nucleic acid. Ninomiya K, Minohata T, Nishimura M, Sisido M.).

Although many methods have been reported for using a stop codon as a codon for introducing unnatural amino acids, the PURE system described above can be used to construct a synthetic system excluding natural amino acids and ARS, so that unnatural amino acids can be introduced instead of the natural amino acids excluded for codons encoding the amino acids (J. Am. Chem. Soc. 2005;127:11727-35. Ribosomal synthesis of unnatural peptides. Josephson K, Hartman MC, Szostak JW.). Furthermore, by decoding the degeneracy of the codon, an unnatural amino acids can be added

without excluding natural amino acids (Kwon I, et al. Breaking the degeneracy of the genetic code. J Am Chem Soc. 2003, 125, 7512-3.). Peptides containing N-methylamino acids can be synthesized in ribosomes by using cell-free translation systems such as PURE system.

Examples of the known display library utilizing cell-free translation systems include, in addition to mRNA displays, a cDNA display in which a complex of a peptide and puromycin is bound to cDNA encoding the peptide (Nucleic Acids Res. 2009;37(16):e108. cDNA display: a novel screening method for functional disulfide-rich peptides by solid-phase synthesis and stabilization of mRNA-protein fusions. Yamaguchi J, Naimuddin M, Biyani M, Sasaki T, Machida M, Kubo T, Funatsu T, Husimi Y, Nemoto N.), a ribosome library utilizing a relatively stable complex of ribosome and a translation product during mRNA translation (Proc Natl Acad Sci U S A. 1994;91:9022-6. An in vitro polysome display system for identifying ligands from very large peptide libraries. Mattheakis LC, Bhatt RR, Dower WJ.), a covalent display in which bacteriophage endonuclease P2A forms a covalent bond with DNA (Nucleic Acids Res. 2005;33:e10. Covalent antibody display--an in vitro antibody-DNA library selection system. Reiersen H, Lobersli I, Loset GA, Hvattum E, Simonsen B, Stacy JE, McGregor D, Fitzgerald K, Welschof M, Brekke OH, Marvik OJ.) and a CIS display utilizing the replication initiation protein RepA of the plasmid of the microorganism to bind to the replication initiation point ori (Proc Natl Acad Sci U S A. 2004;101:2806-10. CIS display: In vitro selection of peptides from libraries of protein-DNA complexes. Odegrip R, Coomber D, Eldridge B, Hederer R, Kuhlman PA, Ullman C, FitzGerald K, McGregor D.). Also known is in vitro compartmentalization in which, for each molecule of DNA that constitutes a DNA library, a transcriptional translation system is encapsulated in a water-in-oil emulsion or liposome, and a translation reaction is performed (Nat Biotechnol. 1998;16:652-6. Man-made cell-like compartments for molecular evolution. Tawfik DS, Griffiths AD.). The display library can be created using an appropriate known method.

The "barcode sequence" (also referred to as barcode) as used herein means a nucleic acid sequence capable of being detected and identified. The barcode sequence can have, for example, 10 to 100 nucleotides, and can be incorporated into a variety of nucleic acids. As a result, in a library, a nucleic acid incorporating a barcode sequence can be identified or grouped by the barcode.

The "barcoded" as used herein means containing a barcode sequence. When the peptide-nucleic acid complex is barcoded, it is possible to identify a library containing a peptide hit in a screening by identifying a barcode sequence using the correspondence between the specific library and the barcode sequence. Then, once the library is identified, it becomes easier to recognize a nucleic acid sequence encoding the peptide that bind easily to a target. The barcode sequence may contain an identification barcode sequence and/or a common barcode sequence, and the barcode sequence may be a combination of two types of an identification barcode sequence and a common barcode sequence. The identification barcode sequence is different for each nucleic acid display library so that from which nucleic acid display library the barcoded peptide-nucleic acid complex containing the barcode sequence is derived can be identified. The common barcode sequence is a barcode sequence common to any nucleic acid display library, and the entire nucleic acid can be amplified by using a primer that anneals to the common barcode sequence if the entire mixture of nucleic acid display libraries is to be amplified by PCR.

Methods for barcoding are not particularly limited, but a barcoded peptide-nucleic acid complex can be obtained by reverse transcribing a nucleic acid sequence using a primer containing a barcode sequence (also referred to as a first barcode primer or a first primer) and a reverse transcriptase to prepare a double-strand of a nucleotide chain of a nucleic acid moiety of the peptide-nucleic acid complex and a nucleotide chain extended with the primer (complementary DNA (cDNA)). The barcode sequence is attached to the 5' end of the cDNA. The obtained double-strand is DNA/mRNA duplex or DNA/DNA duplex, preferably cDNA/mRNA duplex. Since the nucleic acid moiety of the peptide-nucleic acid complex is double-stranded, the stability of the peptide-nucleic acid complex is improved and it is easy to handle the peptide-nucleic acid complex stably under various conditions of amplification, replication, transcription, reverse transcription, purification, or the like. Other methods for barcoding include introducing a barcode during preparation of a nucleic acid (DNA) library or introducing a barcode during linkage (ligation) with puromycin. For example, for a DNA library, PCR is performed with a primer containing a barcode sequence, then transcription is performed to prepare an mRNA containing the barcode sequence. The mRNA is then linked to a peptide with a puromycin linker as shown in Figure 1 of Douthwaite J. A. et al. "Ribosome Display and Related Technologies: Methods and Protocols" (Published from Humana, 2011, pages 113-135), or the mRNA containing the barcode sequence is introduced into a nucleic acid moiety of a puromycin linker and linked to a peptide. A barcoded peptide-nucleic acid complex can then be obtained by translating the mRNA. The nucleic acid is DNA, DNA/mRNA duplex or DNA/DNA duplex, preferably cDNA, cDNA/mRNA duplex or cDNA/cDNA duplex.

The first barcode primer is preferably a primer containing an identification barcode sequence and a common barcode sequence, and the common barcode sequence is preferably located at the 5' end of the identification barcode sequence.

The number of bases of the first barcode primer is not limited as long as it can perform the function of the primer, and may be, for example, 15 to 150, 15 to 140, 15 to 130, 15 to 120, 15 to 110, 15 to 100, 15 to 90, 15 to 80, 15 to 70, 15 to 60, 15 to 50, 15 to 40, 15 to 30, 25 to 150, 25 to 140, 25 to 130, 25 to 120, 25 to 110, 25 to 100, 25 to 90, 25 to 80, 25 to 70, 25 to 60, 25 to 50, 25 to 40, or 25 to 30. The number of bases of the first barcode primer is preferably 25 to 150, more preferably 25 to

100, and particularly preferably 25 to 60.

The number of bases of the identification barcode sequence is not limited as long as it can perform the function of the barcode sequence, and may be, for example, 15 to 30, 15 to 27, 15 to 25, 15 to 22, 15 to 20, 15 to 17, 17 to 30, 17 to 27, 17 to 25, 17 to 22, 17 to 20, 20 to 30, 20 to 27, 20 to 25, 20 to 22, 22 to 30, 22 to 27, 22 to 25, or 25 to 30. The number of bases of the identification barcode sequence is preferably 15 to 30, more preferably 17 to 25, and particularly preferably 17 to 22.

The number of bases of the common barcode sequence is not limited as long as it can perform the function of the barcode sequence, and may be, for example, 15 to 30, 15 to 27, 15 to 25, 15 to 22, 15 to 20, 15 to 17, 17 to 30, 17 to 27, 17 to 25, 17 to 22, 17 to 20, 20 to 30, 20 to 27, 20 to 25, 20 to 22, 22 to 30, 22 to 27, 22 to 25, or 25 to 30. The number of bases of the common barcode sequence is preferably 15 to 30, more preferably 17 to 25, and particularly preferably 17 to 22.

In the present embodiments, template mRNAs are independently translated using cell-free translation systems to prepare a corresponding plurality of nucleic acid display libraries. Even with the same template mRNA, different peptides can be prepared according to the cell-free translation system used in performing the translation. The cell-free translation systems used in preparing the plurality of nucleic acid display libraries contain a different combination of amino acids from one another among the plurality of nucleic acid display libraries. In the present embodiments, different peptides can be prepared even with the same cell-free translation system used in performing the translation when different template mRNAs (e.g., mRNAs with different numbers of repeats of random sequences) are used.

In an aspect, the plurality of nucleic acid display libraries are nucleic acid display libraries, each of which is produced by translation based on a different genetic code table from one another. In an aspect, the plurality of nucleic acid display libraries are nucleic acid display libraries, each of which is produced by translation based on the same genetic code table. The nucleic acid sequence of the template mRNA and the amino acid sequence of the peptide, which is a translation product, depend on the codon table in the cell-free translation system used. In an aspect, correspondence between at least one, two or more, three or more, four or more, five or more, six or more, seven or more, eight or more, nine or more, 10 or more, 11 or more, 12 or more, 13 or more, 14 or more, or 15 or more codons and amino acids is different from each other among a plurality of nucleic acid display libraries. In an aspect, correspondence between 48 or less, 45 or less, 40 or less, 35 or less, 30 or less, 25 or less, 20 or less, or 15 or less codons and amino acids is different from each other among a plurality of nucleic acid display libraries. Correspondence between 1 or more and 48 or less, 1 or more and 45 or less, 1 or more and 40 or less, 1 or more and 35 or less, 1 or more and 30 or less, 1 or more and 25 or less, 1 or more and 20 or less, 2 or more and 48 or less, 2 or more and 40 or less, 2 or more and 35 or less, 2 or more and 30 or less, 2 or more and 25 or less, or 2 or more and 20 or less codons and amino acids is different from each other among a plurality of nucleic acid display libraries. Preferably, correspondence between 1 or more, 48 or less, or 1 or more and 48 or less, more preferably 1 or more, 40 or less, or 1 or more and 40 or less, particularly preferably 2 or more, 30 or less, or 2 or more and 30 or less codons and amino acids is different from each other among a plurality of nucleic acid display libraries.

The number of nucleic acid display libraries provided in the step (1) is at least 2, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, 190 or more, 200 or more, 210 or more, 220 or more, 230 or more, 240 or more, 250 or more, 260 or more, 270 or more, 280 or more, 290 or more, 300 or more, 310 or more, 320 or more, 330 or more, 340 or more, or 350 or more. The number of nucleic acid display libraries provided in the step (1) is 400 or less, 390 or less, 380 or less, 370 or less, 360 or less, 350 or less, 340 or less, 330 or less, 320 or less, 310 or less, 300 or less, 290 or less, 280 or less, 270 or less, 260 or less, 250 or less, 240 or less, 230 or less, 220 or less, 210 or less, 200 or less, 190 or less, 180 or less, 170 or less, 160 or less, 150 or less, 140 or less, 130 or less, 120 or less, 110 or less, 100 or less, 90 or less, 80 or less, 70 or less, 60 or less, 50 or less, 40 or less, 30 or less, 20 or less, or 10 or less. The number of nucleic acid display libraries provided in the step (1) is 2 or more and 400 or less, 2 or more and 200 or less, 2 or more and 100 or less, 2 or more and 50 or less, 2 or more and 30 or less, 2 or more and 15 or less, 2 or more and 10 or less, 3 or more and 400 or less, 3 or more and 200 or less, 3 or more and 100 or less, 3 or more and 50 or less, 3 or more and 30 or less, 3 or more and 15 or less, 3 or more and 10 or less, 4 or more and 400 or less, 4 or more and 200 or less, 4 or more and 100 or less, 4 or more and 50 or less, 4 or more and 30 or less, 4 or more and 15 or less, or 4 or more and 10 or less. Preferably, the number of nucleic acid display libraries provided in the step (1) is 2 or more, 400 or less, or 2 or more and 400 or less, more preferably 2 or more, 100 or less, or 2 or more and 100 or less, particularly preferably 2 or more, 50 or less, or 2 or more and 50 or less, and most preferably 2 or more, 10 or less, or 2 or more and 10 or less.

The nucleic acid display library is a library having a diversity of $10^3$ or more, $10^4$ or more, $10^5$ or more, $10^6$ or more, $10^7$ or more, $10^8$ or more, $10^9$ or more, or $10^{10}$ or more, $10^{11}$ or more, or $10^{12}$ or more. These diversities are not limited to measured values, and are theoretical values. The "diversity" as used herein means the types of peptide-nucleic acid complexes contained in a nucleic acid display library, and the upper limit theoretically depends on the types of random sequences in the template DNAs used.

When determining the "theoretical (total) number of variations" in the peptide-nucleic acid complex contained in the nucleic acid display library of the present disclosure, those that cannot actually be manufactured as the peptide-nucleic acid complex are not included in the theoretical (total) number. For example, in the following cases (1) and (2),

corresponding amino acids are not translationally synthesized, thus peptide-nucleic acid complexes containing such amino acids are not included in the theoretical (total) number: (1) when an amino acid is added to a translation solution as a material for the peptide, but nucleic acids encoding the same amino acid are not contained as a template; (2) when a translation solution used contains a base sequence of nucleic acids that serve as a template for a peptide but not contain a corresponding amino acid for the peptide. In addition, if side reactants or unreacted compounds are present in the process of producing the cyclic peptide compound, those are not included in the theoretical (total) number.

For example, if a codon table in which a set of codon units is corresponds to a single amino acid is used to translationally synthesize a peptide with 11 residues from a base sequence in which the codon units are randomly arranged using a total of 20 natural amino acids, the theoretical number of variations is $20^{10}$ when the translation initiation amino acid is fixed to methionine, which is the upper limit.

The nucleic acid display library is a library containing $10^3$ or more, $10^4$ or more, $10^5$ or more, $10^6$ or more, $10^7$ or more, $10^8$ or more, $10^9$ or more, $10^{10}$ or more, $10^{11}$ or more, or $10^{12}$ or more peptide-nucleic acid complexes.

<u>Step (2)</u>

The step (2) is a step of mixing the plurality of nucleic acid display libraries obtained in the step (1) to prepare a mixed nucleic acid display library.

In the step (2), at least 2, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, 190 or more, 200 or more, 210 or more, 220 or more, 230 or more, 240 or more, 250 or more, 260 or more, 270 or more, 280 or more, 290 or more, 300 or more, 310 or more, 320 or more, 330 or more, 340 or more, or 350 or more nucleic acid display libraries are mixed to prepare a mixed nucleic acid display library. In the step (2), 400 or less, 390 or less, 380 or less, 370 or less, 360 or less, 350 or less, 340 or less, 330 or less, 320 or less, 310 or less, 300 or less, 290 or less, 280 or less, 270 or less, 260 or less, 250 or less, 240 or less, 230 or less, 220 or less, 210 or less, 200 or less, 190 or less, 180 or less, 170 or less, 160 or less, 150 or less, 140 or less, 130 or less, 120 or less, 110 or less, 100 or less, 90 or less, 80 or less, 70 or less, 60 or less, 50 or less, 40 or less, 30 or less, 20 or less, or 10 or less nucleic acid display libraries are mixed to prepare a mixed nucleic acid display library. In the step (2), 2 or more and 400 or less, 2 or more and 200 or less, 2 or more and 100 or less, 2 or more and 50 or less, 2 or more and 20 or less, 2 or more and 15 or less, 2 or more and 10 or less, 3 or more and 400 or less, 3 or more and 200 or less, 3 or more and 100 or less, 3 or more and 50 or less, 3 or more and 20 or less, 3 or more and 15 or less, 3 or more and 10 or less, 4 or more and 400 or less, 4 or more and 200 or less, 4 or more and 100 or less, 4 or more and 50 or less, 4 or more and 20 or less, 4 or more and 15 or less, or 4 or more and 10 or less nucleic acid display libraries are mixed to prepare a mixed nucleic acid display library. In the step (2), preferably 2 or more, 400 or less, or 2 or more and 400 or less, more preferably 2 or more, 100 or less, or 2 or more and 100 or less, particularly preferably 2 or more and 10 or less nucleic acid display libraries are mixed to prepare a mixed nucleic acid display library.

In the step (2), a more diverse library can be prepared from a single template RNA by mixing the plurality of nucleic acid display libraries to prepare a mixed nucleic acid display library. For example, when a single template RNA and 10 cell-free translation systems are used to prepare 10 types of nucleic acid display libraries (assuming that the diversity of each nucleic acid display library is $10^3$) and then the resulting libraries are mixed to prepare a mixed nucleic acid display library, the diversity of the mixed nucleic acid display library is $10^4$. That is, when the screening operation is performed once using the mixed nucleic acid display library, the same results as when the screening operations are separately performed using each of 10 nucleic acid display libraries can be obtained, and thus the screening can be performed very efficiently. In addition, by selecting a different barcode sequence for each library (or cell-free translation system used in its preparation), the barcode sequence can be used as a landmark to identify which library (or cell-free translation system used in its preparation) the peptide-nucleic acid complex is derived from. For example, a mixed nucleic acid display library contains peptide-nucleic acid complexes having different identification barcode sequences and a common barcode sequence, and each identification barcode sequence corresponds to the nucleic acid display library from which the peptide-nucleic acid complex is derived, as shown below.

[Formula 2]

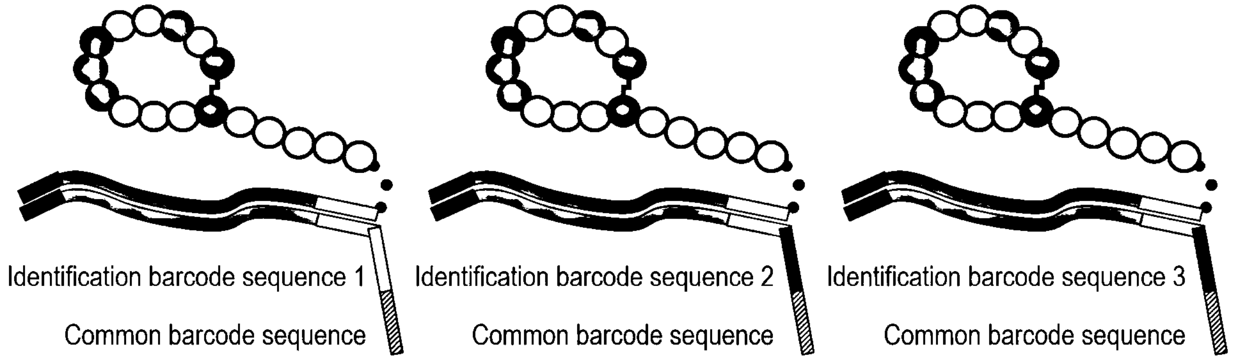


Step (3)

The step (3) is a step of bringing the mixed nucleic acid display library obtained in the step (2) into contact with a target molecule. When the library is brought into contact with the target molecule, the peptide moiety of some barcoded peptide-nucleic acid complexes contained in the library binds to the target molecule. The target molecule may be fixed in a manner well known to those skilled in the art. A peptide-nucleic acid complex that binds to the target molecule (a peptide-nucleic acid complex with a high affinity for the target molecule) can be selected by bringing the mixed nucleic acid display library into contact with the target molecule and washing away the peptide-nucleic acid complex that does not bind to the target molecule (panning method). The step (3) may be repeated multiple times, for example, two to five times. By repeating the step (3), the accuracy of the selection can be improved.

From the thus selected barcoded peptide-nucleic acid complex, a nucleic acid (e.g., cDNA) contained in the nucleic acid moiety is eluted. The nucleic acid contains a barcode sequence derived from a first barcode primer. A nucleic acid (cDNA) corresponding to the nucleic acid moiety may be amplified from the eluted nucleic acid by PCR. The obtained nucleic acid can be amplified by a PCR method and analyzed for the nucleic acid sequence to identify the sequence of the peptide bound to the target molecule. The method according to the present embodiment may further include steps (3A) and/or (3B) between the steps (3) and (4).

Step (3A)

The step (3A) is performed between the steps (3) and (4), and is a step of eluting a nucleic acid (e.g., cDNA) from the barcoded peptide-nucleic acid complex bound to the target molecule. The barcoded peptide-nucleic acid complex can be eluted by treating with a specific enzyme, heating, applying a chemical stimulus, or irradiating with a specific light. Examples of the enzyme that can be used for elution include TEV protease. When the light is irradiated to elute the nucleic acid, it is preferable to irradiate it with the light having a wavelength of 300 nm or more and 500 nm or less. Examples of the chemical stimulus include treating with an acid or base. The eluted nucleic acid contains a barcode sequence derived from the first barcode primer at the 5' end. The eluted nucleic acid may be converted into a double-stranded nucleic acid (e.g., double-stranded cDNA) through primer extension using a forward primer.

Step (3B)

The step (3B) is performed between the steps (3) and (4) (for example, after the step (3A)), and is a step of identifying an amino acid sequence of the peptide moiety of the barcoded peptide-nucleic acid complex bound to the target molecule. The amino acid sequence of the peptide moiety is identified based on a nucleic acid sequence and/or a barcode sequence of the nucleic acid moiety of the barcoded peptide-nucleic acid complex bound to the target molecule. In addition, the barcode sequence of the nucleic acid (e.g., cDNA) eluted in the step (3A) can be used as a landmark to identify the library (or cell-free translation system used in its preparation) from which the peptide-nucleic acid complex is derived. For example, when two types of template mRNA are translated using two types of cell-free translation systems to prepare a total of four nucleic acid display libraries, the chemical structure of the peptide moiety can be theoretically understood from the combination by reverse transcribing using a primer containing a sequence complementary to the barcode sequence corresponding to the cell-free translation system used and identifying the nucleic acid sequence of the nucleic acid moiety of the peptide-nucleic acid complex and the barcode sequence.

Based on the identified amino acid sequence information, a peptide can be synthesized by any method. This peptide can be used to evaluate the binding activity and inhibitory activity against the target molecule, or to evaluate using

cells or animals to obtain a desired peptide.

In the present disclosure, the target molecule used in the screening method are not particularly limited, and examples thereof include proteins, peptides, nucleic acids, sugars, lipids, and the like, but it is preferred that proteins are targeted. In an aspect, a screening method in the present disclosure can obtain a peptide that inhibits a protein-protein interaction (PPI). In particular, it is believed that the surfaces of both proteins involved in PPI contain sites called hot spots, which enhance the binding power of PPI. Examples of the PPI include hydrophobic interactions, electrostatic interactions, van der Waals forces, hydrogen bonds, photo-crosslinks, and salt bridges. Such PPI may result in phosphorylation, conformational changes or localization of proteins, complex formation of proteins with each other (e.g., multisubunitization), and the like, and may be involved in protein modification, transport, folding changes, signaling, and the like. Although not intended to be bound by a particular theory, it is believed that the side chains of the amino acids constituting the peptides contained in the libraries in the present disclosure can inhibit PPI by entering this site. In other words, according to the libraries and screening methods in the present disclosure, it is believed that peptides that inhibit PPI can be obtained regardless of the type of target molecule.

In one non-limiting aspect, the target molecule used in the screening method in the present disclosure is immobilized on a carrier for use. The carrier is not particularly limited if the target molecule can be immobilized, and examples thereof include beads or resins. The target molecule can be fixed to the support by known methods.

The target molecules of the screening methods in the present disclosure are not particularly limited. Since the libraries in the present disclosure include peptide compounds capable of specifically binding to various target molecules, in an aspect, it may enable developing drugs for tough targets that have previously been challenging in drug discovery.

Step (4)

The step (4) is a step of amplifying a nucleic acid or cDNA thereof corresponding to the nucleic acid moiety of the barcoded peptide-nucleic acid complex bound to the target molecule, using a primer containing a sequence complementary to the barcode sequence. The amplified nucleic acid has the same nucleic acid sequence as the nucleic acid contained in the barcoded peptide-nucleic acid complex.

In the step (4), a nucleic acid corresponding to the nucleic acid moiety of the barcoded peptide-nucleic acid complex bound to the target molecule is amplified by a PCR method. First, the nucleic acid corresponding to the nucleic acid moiety is amplified by a PCR method using a forward primer (Fw primer). The Fw primer has a sequence complementary to the nucleic acid sequence of the 5' region of the nucleic acid moiety. When the resulting nucleic acid is amplified by a PCR method using a primer containing a sequence complementary to the common barcode sequence (common barcode primer), the cDNA can be amplified without considering differences in the nucleic acid display library from which the cDNA is derived. This series of reactions can be achieved by simultaneously adding the Fw primer and the common barcode primer and repeating amplification by a PCR method.

[Formula 3]

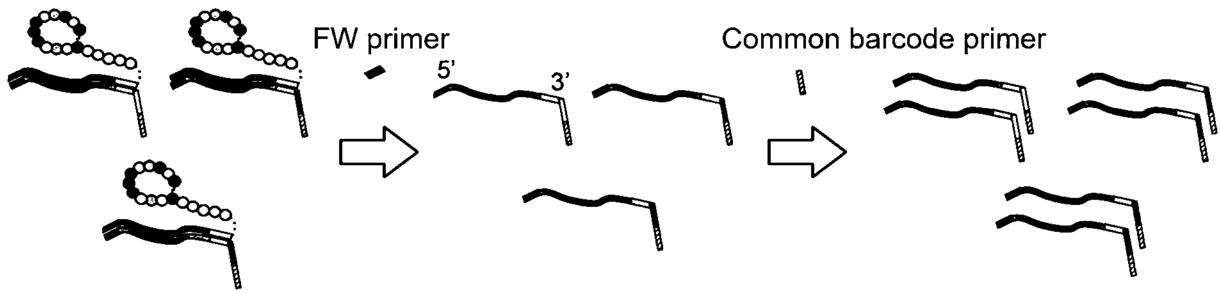


On the other hand, when the nucleic acid corresponding to the nucleic acid moiety is amplified by PCR method using a primer containing a sequence complementary to the identification barcode sequence (also referred to as a second barcode primer or a second primer), the cDNA can be selectively amplified for each nucleic acid display library from which the cDNA is derived, as shown below. In general, nucleic acids being less enriched and nucleic acids being more enriched can be seen in the panning method. When enriched by the panning method, the nucleic acids being less enriched become relatively fewer, so that when a nucleic acid being less enriched is a peptide-nucleic acid complex bound to the target molecule, it may be overlooked. By selectively amplifying according to the identification barcode sequence of the barcoded peptide-nucleic acid complex bound to the target molecule by a PCR method, it is possible to amplify a nucleic acid being less enriched, thereby avoiding missing of a promising peptide.

[Formula 4]

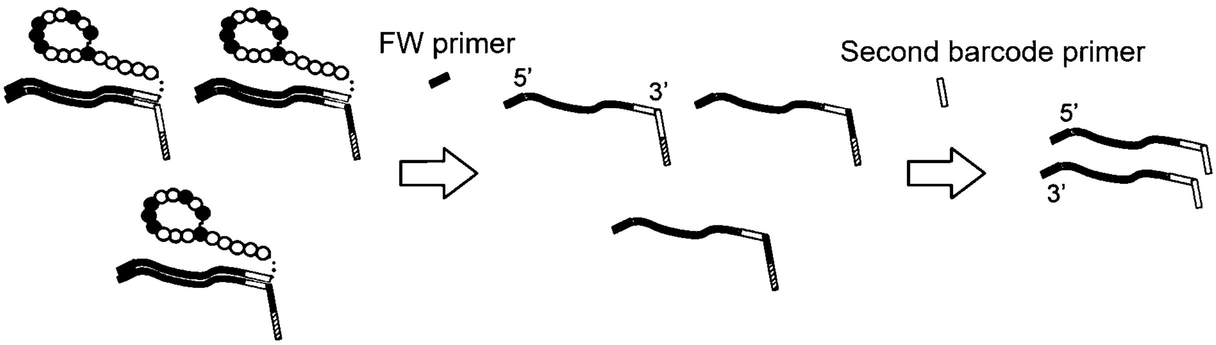


The second barcode primer is preferably a primer containing a sequence complementary to the identification barcode sequence, and may be a primer consisting of a sequence complementary to the identification barcode sequence. The sequence complementary to the identification barcode sequence contained in the second barcode primer is selected such that it corresponds to the nucleic acid display library to be selectively amplified. The second barcode primer may be a primer having 10 to 150, 10 to 140, 10 to 130, 10 to 120, 10 to 110, 10 to 100, 10 to 90, 10 to 80, 10 to 70, 10 to 60, 10 to 50, 10 to 40, 10 to 30, 10 to 20, 15 to 150, 15 to 140, 15 to 130, 15 to 120, 15 to 110, 15 to 100, 15 to 90, 15 to 80, 15 to 70, 15 to 60, 15 to 50, 15 to 40, 15 to 30, 25 to 150, 25 to 140, 25 to 130, 25 to 120, 25 to 110, 25 to 100, 25 to 90, 25 to 80, 25 to 70, 25 to 60, 25 to 50, 25 to 40, or 25 to 30 bases. The second barcode primer may be a primer having preferably 10 to 150, more preferably 10 to 50, and particularly preferably 15 to 30 bases.

In the present embodiments, it is preferable the steps (1) to (4) constitute one cycle (also referred to as "one round"), and the cycle is repeated multiple times. The number of repeats may be, for example, 2 or more cycles, 3 or more cycles, 4 or more cycles, 5 or more cycles, 6 or more cycles, 7 or more cycles, 8 or more cycles, 9 or more cycles, or 10 or more cycles. By repeating the cycle, the nucleic acid of the peptide-nucleic acid complex bound to the target can be enriched.

Step (5)

The method according to the present embodiment may further include a step (5) after the step (4). For example, the step (5) can be performed after the step (4) without starting the next cycle. The step (5) is a step of further amplifying the nucleic acid amplified in the step (4) using a forward primer (Fw primer) and a reverse primer (Rv primer). The nucleic acid amplified in the step (5) does not contain the identification barcode sequence. By amplifying by PCR method using the Fw primer and the Rv primer, the identification barcode sequence can be removed and only the nucleic acid corresponding to the peptide moiety can be amplified.

The Fw primer has a sequence complementary to the nucleic acid sequence of the 5' region of the nucleic acid moiety, and the Rv primer has a sequence complementary to the nucleic acid sequence of the 3' region of the nucleic acid moiety. That is, the Fw primer and the Rv primer are primers capable of annealing to the 5' and 3' regions of the nucleic acid moiety of the peptide-nucleic acid complex, respectively, and do not contain a sequence complementary to the barcode sequence. By the step (5), the nucleic acid corresponding to the peptide moiety can be obtained without the identification barcode sequence, and the next round (a series of steps (1) to (4)) can be performed. If the nucleic acid sequence of the nucleic acid corresponding to the peptide moiety can be identified, a desired peptide can be synthesized by translating the nucleic acid sequence using a cell-free translation system corresponding to the type of the identification barcode sequence.

[Formula 5]

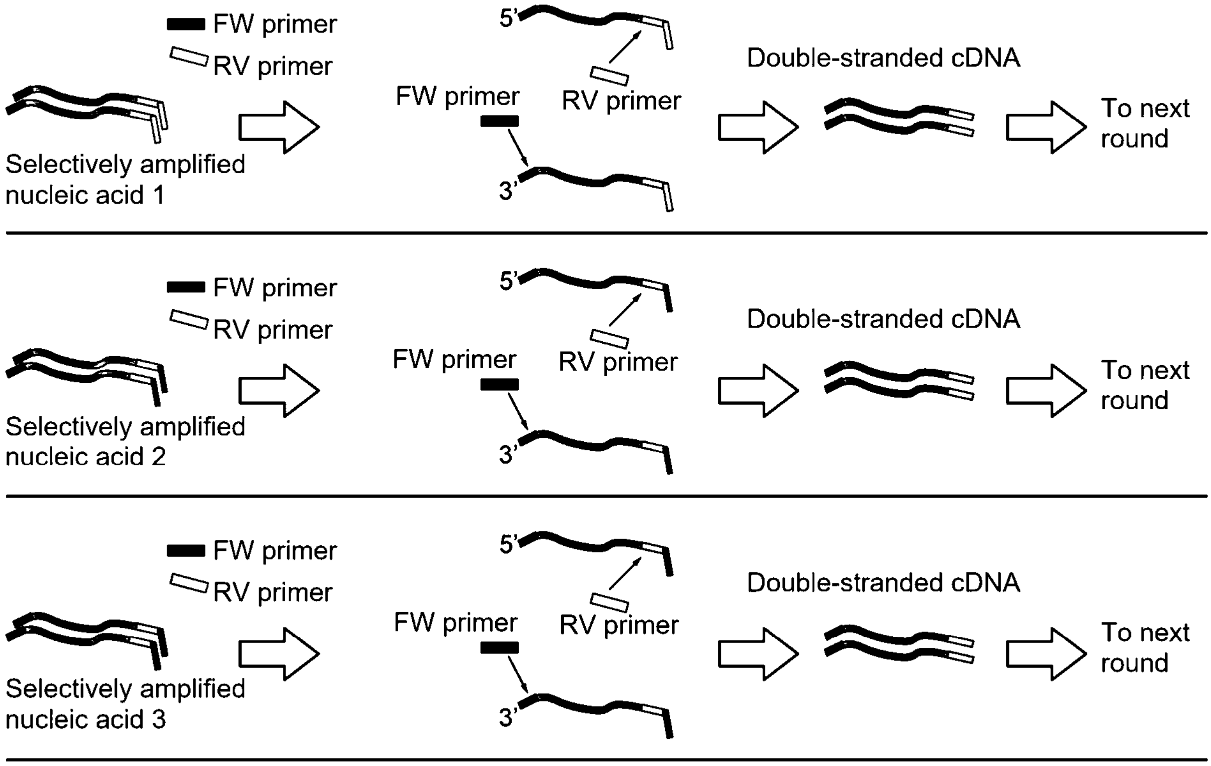


One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(4) amplifying a nucleic acid corresponding to the nucleic acid moiety of the barcoded peptide-nucleic acid complex bound to the target molecule, using a barcode primer; and
(6) repeating the steps (1) to (4) multiple times (cycles).

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(4) amplifying a nucleic acid corresponding to the nucleic acid moiety of the barcoded peptide-nucleic acid complex bound to the target molecule, using a barcode primer; and
(5) removing an identification barcode sequence contained in the nucleic acid moiety of the barcoded peptide-nucleic acid complex bound to the target molecule.

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(4) amplifying a nucleic acid corresponding to the nucleic acid moiety of the barcoded peptide-nucleic acid complex bound to the target molecule, using a barcode primer;
(5) removing an identification barcode sequence contained in the nucleic acid moiety of the barcoded peptide-nucleic acid complex bound to the target molecule; and
(6) repeating the steps (1) to (4) multiple times (cycles).

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule; and
(4) amplifying a nucleic acid corresponding to the nucleic acid moiety of the barcoded peptide-nucleic acid complex bound to the target molecule, using a barcode primer.

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(4) amplifying a nucleic acid corresponding to the nucleic acid moiety of the barcoded peptide-nucleic acid complex bound to the target molecule, using a barcode primer; and
(6) repeating the steps (1) to (4) multiple times (cycles).

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(4) amplifying a nucleic acid corresponding to the nucleic acid moiety of the barcoded peptide-nucleic acid complex bound to the target molecule, using a barcode primer; and
(5) removing an identification barcode sequence contained in the nucleic acid moiety of the barcoded peptide-nucleic acid complex bound to the target molecule.

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(4) amplifying a nucleic acid corresponding to the nucleic acid moiety of the barcoded peptide-nucleic acid complex bound to the target molecule, using a barcode primer;
(5) removing an identification barcode sequence contained in the nucleic acid moiety of the barcoded peptide-nucleic acid complex bound to the target molecule; and
(6) repeating the steps (1) to (4) multiple times (cycles).

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(3B) identifying an amino acid sequence of the peptide moiety of the barcoded peptide-nucleic acid complex bound to the target molecule; and
(4) amplifying a nucleic acid corresponding to the nucleic acid moiety of the barcoded peptide-nucleic acid complex bound to the target molecule, using a barcode primer.

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(3B) identifying an amino acid sequence of the peptide moiety of the barcoded peptide-nucleic acid complex bound to the target molecule;
(4) amplifying a nucleic acid corresponding to the nucleic acid moiety of the barcoded peptide-nucleic acid complex bound to the target molecule, using a barcode primer; and
(6) repeating the steps (1) to (4) multiple times (cycles).

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;

(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(3B) identifying an amino acid sequence of the peptide moiety of the barcoded peptide-nucleic acid complex bound to the target molecule;
(4) amplifying a nucleic acid corresponding to the nucleic acid moiety of the barcoded peptide-nucleic acid complex bound to the target molecule, using a barcode primer; and
(5) removing an identification barcode sequence contained in the nucleic acid moiety of the barcoded peptide-nucleic acid complex bound to the target molecule.

One embodiment of the present invention is a method for screening for a candidate peptide capable of binding to a target molecule, the method comprising the steps of:

(1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
(2) mixing the plurality of nucleic acid display libraries to prepare a mixed nucleic acid display library;
(3) bringing the mixed nucleic acid display library into contact with the target molecule;
(3A) eluting a nucleic acid from the barcoded peptide-nucleic acid complex bound to the target molecule;
(3B) identifying an amino acid sequence of the peptide moiety of the barcoded peptide-nucleic acid complex bound to the target molecule;
(4) amplifying a nucleic acid corresponding to the nucleic acid moiety of the barcoded peptide-nucleic acid complex bound to the target molecule, using a barcode primer;
(5) removing an identification barcode sequence contained in the nucleic acid moiety of the barcoded peptide-nucleic acid complex bound to the target molecule; and
(6) repeating the steps (1) to (4) multiple times (cycles).

One embodiment of the present invention is a method for screening for a candidate peptide capable of binding to a target molecule, the method comprising the steps of:

(1) independently translating a plurality of nucleic acid libraries using a cell-free translation system to prepare a peptide-nucleic acid complex containing a nucleic acid moiety and a peptide moiety;
(1-1) barcoding the peptide-nucleic acid complex to prepare a plurality of nucleic acid display libraries containing the barcoded peptide-nucleic acid complex;
(2) mixing the plurality of nucleic acid display libraries to prepare a mixed nucleic acid display library;
(3) bringing the mixed nucleic acid display library into contact with the target molecule; and
(4) amplifying a nucleic acid contained in the nucleic acid moiety of the barcoded peptide-nucleic acid complex bound to the target molecule, using a barcode primer.

The definition of each term and the description of each step according to the present embodiment can refer to the description in the embodiments described above.

The "nucleic acid library" herein is a collection of nucleic acids that serve as a template for preparing a nucleic acid display library. The nucleic acid contained in the nucleic acid library may be DNA, mRNA, cDNA, or any combination thereof. A preferred nucleic acid library is an mRNA library.

In the present embodiments, a peptide-nucleic acid complex containing a peptide moiety and a nucleic acid moiety can be prepared by translating a plurality of nucleic acid libraries using a cell-free translation system. The nucleic acid moiety of the resulting peptide-nucleic acid complex may be converted to a duplex with cDNA by reverse transcription using a primer. It can also be converted to a barcoded peptide-nucleic acid complex by reverse transcription using a primer containing a barcode sequence. By combining a plurality of cell-free translation systems with a plurality of nucleic acid libraries during preparing the peptide-nucleic acid complex, a display library of a peptide-nucleic acid complex with a variety of patterns can be prepared. Reverse transcription with a primer (first barcode primer) containing a specific barcode sequence associated with a cell-free translation system used allows identification based on the barcode sequence. Even if the obtained multiple nucleic acid display libraries are mixed to prepare a mixed nucleic acid display library, it is possible to identify the library (or cell-free translation system) from which the barcoded peptide-nucleic acid complex is derived, based on the barcode sequence. In an aspect, the nucleic acid library has a different number of repeats from other nucleic acid libraries. In an aspect, the nucleic acid library may contain at least two or more nucleic acids having a different number of

repeats from one another in a single nucleic acid library. In an aspect, the nucleic acid library may be a library consisting of nucleic acids having the same number of repeats in a single nucleic acid library.

The nucleic acid sequence of a nucleic acid contained in the nucleic acid library contains a random sequence. The random sequence herein is a sequence that is different (sequence that is not common) for each nucleic acid sequence and a sequence consisting of arbitrarily selected amino acids and/or amino acid analogs. The random sequence may contain at least one triplet (codon) specifying an unnatural amino acid. The random sequence preferably contains at least one, 2 to 1000, 2 to 750, 2 to 500, 2 to 250, 2 to 100, 2 to 50, 2 to 20, 2 to 18, 2 to 15, 2 to 12, at least 3, 3 to 1000, 3 to 750, 3 to 500, 3 to 250, 3 to 100, 3 to 50, 3 to 18, 3 to 15, 3 to 12, at least 4, 4 to 1000, 4 to 750, 4 to 500, 4 to 250, 4 to 100, 4 to 50, 4 to 18, 4 It is preferable to include 15, 4 to 12, at least 5, 5 to 1000, 5 to 750, 5 to 500, 5 to 250, 5 to 100, 5 to 50, 5 to 18, 5 to 15, or 5 to 12 triplets selected from a plurality of types of triplets. The number of types of triplets is preferably 64 or less, 60 or less, 55 or less, 50 or less, 45 or less, 40 or less, 35 or less, 30 or less, 25 or less, 20 or less, 15 or less, 10 or less, or 5 or less. Each triplet may be the same or different from each other, and may be randomly selected. It is preferable that the peptide moiety of the peptide-nucleic acid complex contains a predetermined amino acid, and the nucleic acid moiety contains a triplet encoding the amino acid.

One embodiment of the present invention is a method for screening for a candidate peptide capable of binding to a target molecule, the method comprising the steps of:

(1) independently translating a plurality of nucleic acid libraries using a single cell-free translation system to prepare a peptide-nucleic acid complex,
(1-1) barcoding the peptide-nucleic acid complex to prepare a plurality of nucleic acid display libraries containing the barcoded peptide-nucleic acid complex;
(2) mixing the plurality of nucleic acid display libraries to prepare a mixed nucleic acid display library;
(3) bringing the mixed nucleic acid display library into contact with the target molecule; and
(4) amplifying a nucleic acid contained in the nucleic acid moiety of the barcoded peptide-nucleic acid complex bound to the target molecule, using a barcode primer.

The definition of each term and the description of each step according to the present embodiment can refer to the description in the embodiments described above.

One embodiment of the present invention is a method for producing a nucleic acid display library, comprising the following steps of:

(a) designing a nucleic acid display library containing a peptide-nucleic acid complex using an analysis result of principal component analysis with a descriptor as an index;
(b) translating a nucleic acid library using a cell-free translation system to prepare a peptide-nucleic acid complex.

In the present embodiments, a nucleic acid display library containing a peptide-nucleic acid complex is designed using a descriptor as an index. A highly diverse library can be designed by performing principal component analysis from a plurality of descriptors and mapping the first principal component and the second principal component to the vector space in two dimensions (plotting the value of the first principal component to the X axis and the value of the second principal component to the Y axis) as shown in Figures 1 to 3. Compound information from the open-source library RDKit (https://www.rdkit.org/) can be used to calculate the descriptor. The descriptor can be adapted, for example, from those provided on the RDKit website (http://rdkit.org/docs/source/rdkit.Chem.html). Examples of the descriptor include, but are not limited to, 'NumValenceElectrons' (Number of valence electrons), 'PEOE _VSA3' (MOE Charge VSA Descriptor 3 ($-0.25 \leq x < -0.20$)), 'fr_Al_OH' (Number of aliphatic hydroxyl groups), 'fr_Al_OH_noTert' (Number of aliphatic hydroxyl groups excluding tert-OH), 'fr_Ar_N' (Number of aromatic nitrogens), 'fr C_O_noCOO' (Number of carbonyl O, excluding COOH), "fr_C_S' (Number of thiocarbonyl), 'fr_HOCCN' (Number of C(OH)CCN-Ctert-alkyl or C(OH)CCNcyclic), 'fr_NH1' (Number of Secondary amines), 'fr_Ndealkylation2' (Number of tert-alicyclic amines (no heteroatoms, not quinine-like bridged N)), 'fr_Nhpyrrole' (Number of H-pyrrole nitrogens), 'fr_SH' (Number of thiol groups), 'fr_alkyl _halide' (Number of alkyl halides), 'fr_allylic_oxid' (Number of allylic oxidation sites excluding steroid dienone), 'fr_aryl_methyl' (Number of aryl methyl sites for hydroxylation), 'fr_azide' (Number of azide groups), 'fr_azo' (Number of azo groups), 'fr_bicyclic' (Bicyclic), 'fr_diazo' (Number of diazo groups), 'fr_dihydropyridine' (Number of dihydropyridines), 'fr_epoxide' (Number of epoxide rings), 'fr_ether' (Number of ether oxygens (including phenoxy)), 'fr_furan' (Number of furan rings), 'fr_halogen' (Number of halogens), 'fr_hdrzine' (Number of hydrazine groups), 'fr_hdrzone' (Number of hydrazone groups), 'fr_imidazole' (Number of imidazole rings), 'fr_imide' (Number of imide groups), 'fr_isocyan' (Number of isocyanates), 'fr_isothiocyan' (Number of isothiocyanates), 'fr_ketone' (Number of ketones), 'fr_ketone_Topliss' (Number of ketones excluding diaryl, a,b-unsat. dienones, heteroatom on Calpha), 'fr_lactam' (Number of beta lactams), 'fr_lactone' (Number of cyclic esters (lactones)), 'fr_methoxy' (Number of methoxy groups -OCH3), 'fr_morpholine' (Number of morpholine rings), 'fr_nitrile' (Number of nitriles), 'fr_nitro' (Number of nitro groups), 'fr_nitro_arom' (Number of nitro benzene ring substituents),

’fr_nitro_arom_nonortho’ (Number of non-ortho nitro benzene ring substituents), ’fr_nitroso’ (Number of nitroso groups, excluding NO2), ’fr_oxazole’ (Number of oxazole rings), ’fr_phenol’ (Number of phenols), ’fr_phenol_noOrthoHbond’ (Number of phenolic OH excluding ortho intramolecular Hbond substituents), ’fr_phos_acid’ (Number of phosphoric acid groups), ’fr_phos_ester’ (Number of phosphoric ester groups), ’fr_piperdine’ (Number of piperdine rings), ’fr_piperzine’ (Number of piperzine rings), ’fr_priamide’ (Number of primary amides), ’fr_prisulfonamd’ (Number of primary sulfonamides), ’fr_pyridine’ (Number of pyridine rings), ’fr_quatN’ (Number of quaternary nitrogens), ’fr_sulfide’ (Number of thioether), ’fr_sulfonamd’ (Number of sulfonamides), ’fr_sulfone’ (Number of sulfone groups), ’fr_term_acetylene’ (Number of terminal acetylenes), ’fr_tetrazole’ (Number of tetrazole rings), ’fr_thiazole’ (Number of thiazole rings), ’fr_thiocyan’ (Number of thiocyanates), and ’fr_thiophene’ (Number of thiophene rings). This method allows to design libraries with little structural overlap of the peptide-nucleic acid complexes contained in the nucleic acid display libraries among the plurality of nucleic acid display libraries.

The definition of each term and the description of each step according to the present embodiment can refer to the description in the embodiments described above.

[Examples]

Example 1 Panning with plurality of PURE systems using Split & Pool technology

Using peptide display libraries translated with PURE system (hereinafter also referred to as PURE), which is a reconstituted cell-free protein synthesis system derived from a plurality of prokaryotes, mRNA display panning was performed to verify whether a variety of peptide sequence groups that bind to a target protein could be obtained by the present method.

In the first round of panning, peptide display libraries translated with four types of PURE were independently selected, and the selection libraries were amplified by PCR reaction.

At the second round and thereafter, the four types of selection libraries obtained in the previous round were translated with each PURE to prepare peptide displays using four types of reverse transcription primers corresponding to each PURE used. Then, selection was performed with a mixed library of these libraries, and then the selection libraries corresponding to each PURE were independently amplified by PCR using four types of primers, and this procedure was repeated.

Example 1-1: Preparation of panning target protein

Glutathione S-transferase (GST) which was expressed and purified with E. coli was used as the target protein for panning. GST-HisTEV-Bio was prepared from GST-HisTEVAvi with a His tag, a TEV protease cleavage tag, and a biotinylase recognition tag added to the C-terminus according to the method described in WO 2022/138892.

Example 1-2: Preparation of peptide display library

Example 1-2-1: Synthesis of acylated tRNA for use in translation

PUREs used in this panning are defined as PURE1, PURE2, PURE3, and PURE4. Acylated tRNA to be used for these was prepared according to the method described in WO 2018/143145 and WO 2018/225864.

In PURE 1, 19 kinds of amino acids consisting of Asp(SMe), Phe(3-Cl), Hph(3-Cl), MeAla(3-Pyr), MeGly, MeHnl(7-F2), MeHph, MeSer(nPr), MeSer(tBuOH), Nle, Pro(4-pip-4-F2), Pic(2), Ser(3-F-5-Me-Pyr), Ser(NtBu-Aca), Ser(Ph-2-Cl), Ser(iPen), cisPro(4-pip-4-F2), D-MeSer, and nBuGly were used to prepare an Elongator aminoacylated tRNA mixture.

In PURE2, 23 kinds of amino acids including Asp(SMe), MeAla, MeGly, MeHnl(7-F2), MeSer(nPr), MeSer(t-BuOH), MeVal, Nle, Pro(4-pip-4-F2), Pic(2), Ser(NtBu-Aca), and D-MeSer were used to prepare an Elongator aminoacylated tRNA mixture.

In PURE3, 21 kinds of amino acids including Asp(SMe), MeAla(3-Pyr), MeGly, MeHnl(7-F2), MeSer(nPr), MeSer(tBuOH), Nle, Pic(2), cisPro(4-pip-4-F2), and nBuGly were used to prepare an Elongator aminoacylated tRNA mixture.

In PURE4, 23 kinds of amino acids including Asp(SMe), MeAla, MeGly, MeHnl(7-F2), MeSer(nPr), MeSer(t-BuOH), MeVal, Nle, Pro(4-pip-4-F2), Pic(2), cisPro(4-pip-4-F2), and nBuGly were used to prepare an Elongator aminoacylated tRNA mixture.

The final concentration of each acylated tRNA in the translation solution was 10 $\mu$M to 20 $\mu$M. Pnaz-protected pCpA amino acids were subjected to a phenol extraction and thereafter processes without deprotection. Initiator aminoacylated tRNA was the same compound as Acbz-MeCys(StBu)-tRNAfMetCAU described in WO 2018/225864,

and added to the translation solution so that the final concentration was 25 μM.

Relationships between the abbreviations of amino acids illustrated herein and the structures thereof are shown in Table 1.

[Table 1]

| Abbreviation | Amino acid structure | Abbreviation | Amino acid structure |
|---|---|---|---|
| Asp(SMe) | 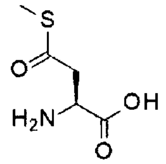 | nBuGly | 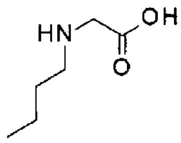 |
| D-MeSer | 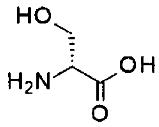 | Nle | 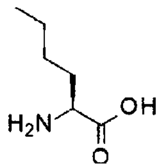 |
| Hph(3-Cl) | 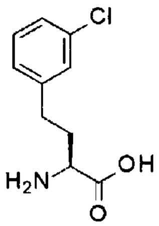 | Phe(3-Cl) | 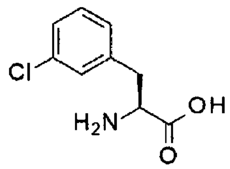 |
| MeAla(3-Pyr) | 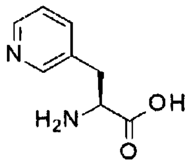 | Pic(2) | 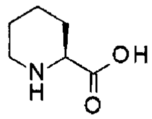 |
| MeGly | 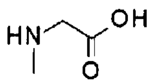 | Pro(4-pip-4-F2) | 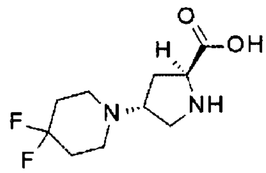 |
| MeHnl(7-F2) | 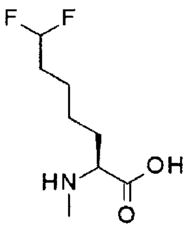 | cisPro(4-pip-4-F2) | 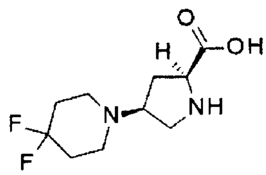 |
| MeHph | 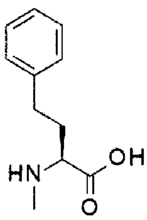 | Ser(3-F-5-Me-Pyr) | 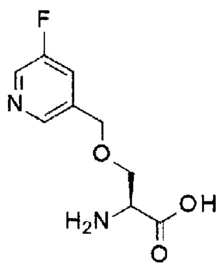 |

(continued)

| Abbreviation | Amino acid structure | Abbreviation | Amino acid structure |
|---|---|---|---|
| MeSer(nPr) | 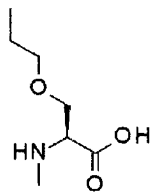 | Ser(iPen) | 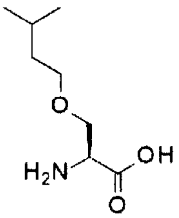 |
| MeSer(tBuOH) | 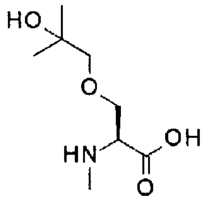 | Ser(NtBu-Aca) | 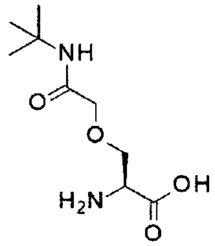 |
| MeVal | 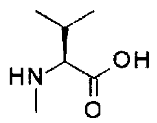 | Ser(Ph-2-Cl) | 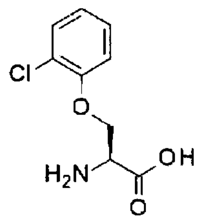 |

Example 1-2-2: Randomized double-stranded DNA library encoding peptide library

The DNA library was constructed in accordance with the method described in WO 2013/100132. In the prepared DNA library, 26 triplets of TTT, TTG, CTT, CTG, ATT, ATG, GTT, GTG, TCT, TCG, CCG, ACT, GCT, TAC, CAT, CAG, AAC, GAT, GAG, TGC, TGG, CGT, CGG, AGT, AGG, and GGT appeared randomly 8 or 9 times (SEQ ID NOs: 1 and 2). Using these, mRNA-puromycin linker ligation products were prepared for use. In the sequences below, each "N" means any one of adenine, cytosine, guanine, and thymine, and there are 8 or 9 triplets represented by "NNN".

DNA Library (SEQ ID NO: 1)

GTAATACGACTCACTATAGGGTTAACTTTAATAAGGAGATATAAATATG$(NNN)_8$TAG CCGACCGGCACCGGCACCGGCAAAAAAA

DNA Library (SEQ ID NO: 2)

GTAATACGACTCACTATAGGGTTAACTTTAATAAGGAGATATAAATATG$(NNN)_9$TAG CCGACCGGCACCGGCACCGGCAAAAAAA

Example 1-2-3: Translation and cyclization of peptide display library

The translation system used was PURE system, which is a reconstituted cell-free protein synthesis system derived from a prokaryotic organism. Specifically, in the four types of PUREs, PURE1 to 4, 1 mM GTP, 1 mM ATP, 20 mM creatine phosphate, 50 mM HEPES-KOH pH 7.6, 100 mM potassium acetate, 2-4 mM magnesium acetate, 2 mM spermidine, 1 mM dithiothreitol, 1 mg/mL E. coli MRE 600 (RNase-negative) derived tRNA (manufactured by F. Hoffmann-La Roche, Ltd.) (some tRNAs were removed by the method described in Nucleic Acids Research, 2010, Vol. 38, No. 6 e89), 4 μg/mL creatine kinase, 6.72 U/mL myokinase, 2 units/mL inorganic pyrophosphatase, 1.1 μg/mL nucleoside diphosphate kinase, 0.26 μM EF-G, 2.7 μM IF1, 0.4 μM IF2, 1.5 μM IF3, 40 μM EF-Tu, 59 μM EF-Ts, 1.2 μM ribosome, 1 μM GlyRS, 0.04-0.4 μM IleRS, 0.16 μM ProRS, 0.09 μM ThrRS, 0.11 μM LysRS, 3 μM in vitro transcribed E. coli tRNA AlaIB, 250 μM glycine, 10-100 μM isoleucine, 250 μM proline, 250 μM threonine, 250 μM lysine, Elongator aminoacylated tRNA

mixture, 25 μM Initiator aminoacylated tRNA (WO 2020/138336 A1), 0.5 μM Penicillin G Amidase (PGA), 1 μM EF-P-Lys, 0.4 units/μL RNasin (R) Ribonuclease Inhibitor (Promega Corporation), and mRNA-puromycin linked construct prepared according to WO 2013/100132 from the double-stranded DNA library described above were added to the translation reaction mixture to be 1 μM.

In PURE1, in addition to the above, 0.5 μM variant PheRS (WO 2016/148044), 1.37 μM AlaRS, 1 μM variant ValRS (WO 2016/148044), 1 μM variant SerRS (WO 2016/148044), 5 mM N-methylphenylalanine, 5 mM N-methylvaline, 5 mM N-methylserine, and 2.5 mM N-methylalanine were added. In PURE3, in addition to the above, 2.73 μM AlaRS, 1 μM variant ValRS (WO 2016/148044), 5 mM N-methylvaline, and 5 mM N-methylalanine were added. After mixing the above factors, the mixture was left still at 37°C for 1 hour to perform the translation.

Example 1-3: Panning

Panning was performed using the translated display library for five rounds in accordance with WO 2013/100132.

Example 1-3-1: Reverse transcription of display library

In the first round, 3 μM Rv primer 1-1 (SEQ ID NO: 3), 50 mM TrisHCl (pH 8.3), 75 mM KCl, 3 mM $MgCl_2$, 0.5 mM dGTP, 0.5 mM dATP, 0.5 mM dCTP, 0.5 mM dTTP, and 8 U/μL M-MLV Reverse transcriptase (H-) (Promega Corporation, M368B) were added to a total of four types of 100 μL display library solutions, which were cyclized, desulfurized, and purified, and made up to 125 μL solution with Nuclease free water, and kept at 42°C for 60 minutes to perform the reverse transcription reaction. To this reverse transcription solution, a buffer of 1 x TBS and 2 mg/mL BSA (invitrogen) was added to prepare a 0.6 μM display library.

At the second round and thereafter, RT primer 1 (SEQ ID NO: 4), RT primer 2 (SEQ ID NO: 5), RT primer 3 (SEQ ID NO: 6), and RT primer 4 (SEQ ID NO: 7) were used for the display library that was translated, cyclized, desulfurized, and purified in PURE1, the PURE2 translation product, the PURE3 translation product, and the PURE4 translation product, respectively, as reverse transcription primers. Specifically, a 3 μM reverse transcription primer corresponding to the type of PURE, 50 mM TrisHCl (pH 8.3), 75 mM KCl, 3 mM $MgCl_2$, 0.5 mM dGTP, 0.5 mM dATP, 0.5 mM dCTP, 0.5 mM dTTP, and 8 U/μL M-MLV Reverse transcriptase (H-) (Promega Corporation, M368B) were added to 24 μL display library solution, which was translated, cyclized, desulfurized, and purified, and made up to 30 μL solution with Nuclease free water, and kept at 42°C for 60 minutes to perform the reverse transcription reaction. To this reverse transcription solution, a buffer of 1 x TBS and 2 mg/ml BSA (invitrogen) was added to prepare a 0.25 μM display library. Thereafter, the display libraries produced by translation of each of the four types of PURE were mixed to form a single display library solution.

Rv primer 1-1 (SEQ ID NO: 3)
TTTTTTTgccggtgccggtgccggtCGG
RT primer 1 (SEQ ID NO: 4)
AAACACGTGGCAAACATTCCAAGAATTACTGACCCCTCGGTTTTTTTgccggtgccggtg
RT primer 2 (SEQ ID NO: 5)
AAACACGTGGCAAACATTCCAAAGCACTCTTAGGCCTCTGTTTTTTTgccggtgccggtg
RT primer 3 (SEQ ID NO: 6)
AAACACGTGGCAAACATTCCAAGGACTGCATACCAGGTTGTTTTTTTgccggtgccggtg
RT primer 4 (SEQ ID NO: 7)
AAACACGTGGCAAACATTCCAAGGCCCAGAAGGATACAACTTTTTTTgccggtgccggtg

Example 1-3-2 Panning with four types of PURE libraries

In Round 1, GST-HisTev-Bio was fixed on Streptavidin coated magnetic beads to be 0.4 μM, and then separately added to four types of display libraries, and reacted at 4°C for 1 hour. After the reaction, the beads were magnetically collected, subjected to supernatant removal, and washed several times with tTBS (manufactured by NACALAI TESQUE, INC.), and then the beads were treated with TEV protease that recognizes and cleaves the TEV protease recognition sequence to elute the nucleic acid.

Specifically, 1 x TEV buffer and 10 mM DTT, 0.1 U/μL AcTEV protease (Thermo Fisher Scientific, Inc., #12575015) were added as a TEV eluate to the washed beads, and reacted. After the reaction, the supernatant was recovered and PCR was performed with library recognition primers (Fw primer (SEQ ID NO: 8), Rv primer 1-2 (SEQ ID NO: 9)) to obtain an input library for the next round.

In addition, qPCR was performed using a portion of the TEV elution product, and the recovery rate of the library for each round was evaluated. For the primers of qPCR, the aforementioned primers (Fw primer (SEQ ID NO: 8), Rv primer 1-3 (SEQ ID NO: 10)) were used. For the qPCR reaction solution, 1 x Ex taq buffer, 0.2 mM dNTP, 0.5 μM Fw primer, 0.5 μM

Rv primer 1-2 or 1-3, 200,000 times dilution SYBR Green I (manufactured by Lonza K.K., catalog# 50513), and 0.02 U/μL Ex Taq polymerase (manufactured by Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

Fw primer (SEQ ID NO: 8)
GTAATACGACTCACTATAGGGTTAACTTTAATAAGGAG
Rv primer 1-2 (SEQ ID NO: 9)
TTTTTTTgccggtgccggtgccggtCGGCTA
Rv primer 1-3 (SEQ ID NO: 10)
TTTGTGGTgccggtgccggtgccggtCGG

In Rounds 2 to 4, GST-HisTev-Bio was fixed on Streptavidin coated magnetic beads to be 0.4 μM, and then added to a mixed solution of four display libraries, and reacted at 4°C for 1 hour. After the reaction, the beads were magnetically collected, subjected to supernatant removal, and washed several times with tTBS (NACALAI TESQUE, INC.) as in Round 1, and then the beads were treated with TEV protease that recognizes and cleaves the TEV protease recognition sequence to elute the nucleic acid. After the reaction, the supernatant was recovered and PCR was performed with library recognition primers (Fw primer (SEQ ID NO: 8), Rv primer 2 (SEQ ID NO: 11)).
Rv primer 2 (SEQ ID NO: 11)
AAACACGTGGCAAACATTCC

The PCR product was subjected to PCR with an Fw primer (SEQ ID NO: 8) and Rv primer 3 (SEQ ID NO: 12), Rv primer 4 (SEQ ID NO: 13), Rv primer 5 (SEQ ID NO: 14) or Rv primer 6 (SEQ ID NO: 15) to obtain four types of PCR products derived from each PURE.

Rv primer 3 (SEQ ID NO: 12)
AAGAATTACTGACCCCTCGG

Rv primer 4 (SEQ ID NO: 13)
AAAGCACTCTTAGGCCTCTG

Rv primer 5 (SEQ ID NO: 14)
AAGGACTGCATACCAGGTTG

Rv primer 6 (SEQ ID NO: 15)
AAGGCCCAGAAGGATACAAC

After that, the four types of PCR products were independently subjected to PCR with primers (Fw primer (SEQ ID NO: 8), Rv primer 1-2 (SEQ ID NO: 9)) to obtain input libraries for the next round. PURE1, PURE2, PURE3, and PURE4 were used for ones derived from Rv primer 3, Rv primer 4, Rv primer 5, and Rv primer 6, respectively, to perform next round of translation.

In addition, qPCR was performed using a portion of the TEV elution product, and the recovery rate of each library for each round was evaluated. For the primers of qPCR, the aforementioned primers (Fw primer (SEQ ID NO: 8) and Rv primer 3 (SEQ ID NO: 12), Rv primer 4 (SEQ ID NO: 13), Rv primer 5 (SEQ ID NO: 14) or Rv primer 6 (SEQ ID NO: 15)) were used. For the qPCR reaction solution, 1 x Ex taq buffer, 0.2 mM dNTP, 0.5 μM Fw primer, 0.5 μM Rv primer, 200,000 times dilution SYBR Green I (manufactured by Lonza K.K., catalog# 50513), and 0.02 U/μL Ex Taq polymerase (manufactured by Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

To draw a calibration curve to estimate the recovery rate of the library, the input library was diluted to be 1E + 8/μL, 1E + 6/μL, and 1E + 4/μL, and subjected to qPCR under the same conditions.

At the second round and thereafter, the input library was divided into two, and selection was made with one in the presence of the target protein and the other in the absence of the target protein, and the nucleic acid recovery rates of both were evaluated from the results of qPCR. The recovery rate (%) of each round of panning was calculated by the expression below. The results are shown in Table 2.

Recovery rate of library (%) = output nucleic acid amount (number of molecules) of library/(input nucleic acid amount (number of molecules) of library)) × 100

[Table 2]

| Round | Translation PURE | Input nucleic acid amount (molecules) of library | In presence of target protein | | In absence of target protein | |
|---|---|---|---|---|---|---|
| | | | Output nucleic acid amount (molecules) | Recovery rate (%) | Output nucleic acid amount (molecules) | Recovery rate (%) |
| **1** | **PURE1** | **1.11.E+14** | **2.34.E+08** | **2.11.E-04** | **-** | **-** |
| **2** | | **3.35.E+11** | **3.59.E+06** | **1.07.E-03** | **3.04.E+05** | **9.07.E-05** |
| **3** | | **3.66.E+11** | **2.82.E+08** | **7.72.E-02** | **5.90.E+06** | **1.61.E-03** |
| **4** | | **4.16.E+11** | **3.41.E+09** | **8.19.E-01** | **1.37.E+06** | **3.30.E-04** |
| **1** | **PURE2** | **1.08.E+14** | **3.37.E+08** | **3.10.E-04** | **-** | **-** |
| **2** | | **4.22.E+11** | **1.75.E+06** | **4.14.E-04** | **4.32.E+05** | **1.02.E-04** |
| **3** | | **3.90.E+11** | **7.92.E+07** | **2.03.E-02** | **2.64.E+06** | **6.79.E-04** |
| **4** | | **7.74.E+11** | **4.47.E+09** | **5.78.E-01** | **1.20.E+06** | **1.55.E-04** |
| **1** | **PURE3** | **9.87.E+13** | **1.75.E+08** | **1.77.E-04** | **-** | **-** |
| **2** | | **2.58.E+11** | **5.40.E+05** | **2.09.E-04** | **4.54.E+04** | **1.76.E-05** |
| **3** | | **3.45.E+11** | **2.43.E+08** | **7.06.E-02** | **3.37.E+06** | **9.77.E-04** |
| **4** | | **7.37.E+11** | **4.88.E+09** | **6.61.E-01** | **1.17.E+06** | **1.59.E-04** |
| **1** | **PURE4** | **1.08.E+14** | **3.69.E+08** | **3.41.E-04** | **-** | **-** |
| **2** | | **2.28.E+11** | **9.10.E+05** | **3.99.E-04** | **3.47.E+04** | **1.52.E-05** |
| **3** | | **2.42.E+11** | **5.75.E+07** | **2.37.E-02** | **1.64.E+06** | **6.76.E-04** |
| **4** | | **5.51.E+11** | **3.18.E+09** | **5.76.E-01** | **1.15.E+06** | **2.09.E-04** |

As a result, in the second half of the rounds, as shown in Table 2, there was a difference in the recovery rate between the selections in the presence of the target protein and in the absence of the target protein. It is thus likely that molecules that are only recovered in the presence of the target protein are present in the library of each round, and they are enriched each time the round is repeated.

Example 1-3-3 Analysis of enrichment sequence

The base sequences of the DNA pools in each round of panning were analyzed, and the analysis of enrichment sequence was performed.

A sequence in which the 11th residue of the peptide produced by translation in the DNA pool selected in the presence of the target protein was Asp (SMe), and the frequency of occurrence was more than 0.05% in at least one round, and the frequency of occurrence increased 10 times or more when a target was added compared to when the pool of the previous round was divided and the target was not added in the panning was extracted as a sequence that was enriched only in the presence of the target protein.

In this analysis, the characteristics of the enrichment sequence were evaluated using principal component analysis. In the principal component analysis, 60 descriptors (shown below) were calculated from each sequence, and after standardizing each value, they were mapped to a vector space with the horizontal axis for the first principal component and the vertical axis for the second principal component. Compound information from the open-source library RDKit (https://www.rdkit.org/) was used to calculate the descriptors.

Names of 60 descriptors

'NumValenceElectrons', 'PEOE_VSA3', 'fr_Al_OH', 'fr_Al_OH_noTert', 'fr_Ar_N', 'fr_C_O_noCOO', 'fr_C_S', 'fr_HOCCN', 'fr_NH1', 'fr_Ndealkylation2', 'fr_Nhpyrrole', 'fr_SH', 'fr_alkyl_halide', 'fr_allylic_oxid', 'fr_aryl_methyl', 'fr_azide', 'fr_azo', 'fr_bicyclic', 'fr_diazo', 'fr_dihydropyridine', 'fr_epoxide', 'fr_ether', 'fr_furan', 'fr_halogen', 'fr_hdrzine', 'fr_hdrzone', 'fr_imidazole', 'fr_imide', 'fr_isocyan', 'fr_isothiocyan', 'fr_ketone', 'fr_ketone_Topliss', 'fr_lactam', 'fr_lactone', 'fr_methoxy', 'fr_morpholine', 'fr_nitrile', 'fr_nitro', 'fr_nitro_arom', 'fr_nitro_arom_nonortho', 'fr_nitroso', 'fr oxazole', 'fr_phenol', 'fr_phenol_noOrthoHbond', 'fr_phos_acid', 'fr_phos_ester', 'fr_piperdine', 'fr_piperzine', 'fr_priamide', 'fr_prisulfonamd', 'fr_pyridine', 'fr_quatN', 'fr_sulfide', 'fr_sulfonamd', 'fr_sulfone', 'fr_term_acetylene', 'fr_tetrazole', 'fr_thiazole', 'fr_thiocyan', and 'fr_thiophene'

As comparison groups to sequences enriched only in the presence of the target protein, 1000 sequences of 11-residue peptide random sequences capable of being synthesized from each combination of the four codon tables, PURE1, PURE2, PURE3, and PURE4, were generated. Figure 1 is a plot of the values of each principal component of the random sequences in each codon table (Random1: PURE1, Random2: PURE2, Random3: PURE3, Random4: PURE4). Figure 2 is a plot of the values of each principal component of the enrichment sequence in each codon table. Figure 3 is a plot of the values of each principal component of enrichment sequences (shown in black) and all random sequences (shown in gray, where each shape corresponds to the following cell-free translation system: ○: PURE1, △: PURE2, □: PURE3, ×: PURE4).

Since each numeric vector in Figures 1 to 3 is a vector representing the characteristic of an actually existing amino acid sequence, the amino acid sequences resembling each other are expressed as similar vectors.

In Figure 1, most of the values of each sequence are close among the four types of random sequences, but some values are unique to the sequences generated from a single codon table. In other words, it can be confirmed that although the random sequences generated from the four types of codon tables are generally close to corresponding amino acid sequences to each other, there are also amino acid sequences generated from only one codon table.

In Figures 2 and 3, the values of the random sequences and each sequence were close in the enrichment sequences, but the values in some enrichment sequences were close to the values distributed only in the specific codon table. In other words, the enrichment sequences were close to the amino acid sequence generated from the four types of codon tables, and also reflected the characteristics of the sequence generated from the four types of codon tables, and it could be confirmed that some peptides have different characteristics in sequences as the results of using a plurality of PURE. From this, it was confirmed that panning with a plurality of PURE systems using the Split & Pool technique enriches peptides with a variety of amino acid sequences that cannot be obtained from one codon table alone.

## Example 2 Panning with plurality of libraries using Split & Pool technique

Verification was performed by mRNA display panning to determine whether a variety of peptide sequence groups that bind to a target protein can be obtained by the present method using a peptide display library with different lengths of random sequences. In addition, mRNA display panning was performed using a library obtained by mixing libraries with different lengths of random sequences in advance without using the present method, and the results of this were taken as a comparative basis.

In the panning with the Split & Pool technique, translation was performed using four types of libraries with different lengths and one type of PURE, and four types of reverse transcription primers corresponding to the length of each library were used to prepare peptide display libraries. Then, selection was performed with a mixed library of these libraries, and then the selection libraries corresponding to each PURE were independently amplified by PCR using four types of primers, and this procedure was repeated.

In the panning under comparison conditions, four types of libraries were mixed and then translated with one type of PURE, and a peptide display library was prepared using one type of reverse transcription primer. Then, selection and PCR were performed, and these procedures were repeated.

### Example 2-1: Preparation of panning target protein

Glutathione S-transferase (GST) which was expressed and purified with E. coli was used as the target protein for panning. GST-HisTEV-Bio was prepared from GST-HisTEVAvi with a His tag, a TEV protease cleavage tag, and a biotinylase recognition tag added to the C-terminus according to the method described in WO 2022/138892.

### Example 2-2: Preparation of peptide display library

#### Example 2-2-1: Synthesis of acylated tRNA for use in translation

Acylated tRNA to be used for these was prepared according to the method described in WO 2018/143145 and WO 2018/225864. 19 kinds of amino acids including Asp(SMe), Phe(3-Cl), Hph(3-Cl), MeAla(3-Pyr), MeGly, MeHnl(7-F2), MeHph, MeSer(nPr), MeSer(tBuOH), Nle, Pro(4-pip-4-F2), Pic(2), Ser(3-F-5-Me-Pyr), Ser(NtBu-Aca), Ser(Ph-2-Cl), Ser(iPen), cisPro(4-pip-4-F2), D-MeSer, and nBuGly were used to prepare an Elongator aminoacylated tRNA mixture. The final concentration of each acylated tRNA in the translation solution was 10 μM to 20 μM. Pnaz-protected pCpA amino acids were subjected to a phenol extraction and thereafter processes without deprotection. Initiator aminoacylated tRNA was the same compound as Acbz-MeCys(StBu)-tRNAfMetCAU described in WO 2018/225864, and added to the translation solution so that the final concentration was 25 μM.

Example 2-2-2: Randomized double-stranded DNA library encoding peptide library

As template DNA, synthetic DNA oligos were prepared in which triplets of NNB appeared 5, 7, 9 or 11 times repeatedly at random (IDT, Inc.) (SEQ ID NOs: 16, 17, 18, 19). In the following sequence, "N" means any one of four DNA bases A, T, G, and C, and each "N" may be the same or different from each other. "B" means any one of three DNA bases, T, G, and C, and each "B" may be the same or different from each other. Four double-stranded DNA libraries (SEQ ID NOs: 21-24) were prepared by mixing each of these 0.01 μM synthetic oligo and 1 μM synthetic oligo DNA 1 (SEQ ID NO: 20), Rv primer 1-2 (SEQ ID NO: 9), 1 x PrimeSTAR Buffer, 0.2 mM dNTP, and 0.025 U/μL PrimeSTAR HS DNA polymerase (Takara Bio Inc.), heating at 95°C for 2 minutes, and then repeating the cycles of 95°C for 10 seconds, 57°C for 20 seconds, and 72°C for 30 seconds for 10 cycles. Using these, mRNA-puromycin linker ligation products were prepared for use in accordance with the method described in WO 2013/100132.

7-residue DNA oligo (SEQ ID NO: 16)
GAGATATAAATATG$(NNB)_5$TAGCCGACCGGCACCGGC
9-residue DNA oligo (SEQ ID NO: 17)
GAGATATAAATATG$(NNB)_7$TAGCCGACCGGCACCGGC
11-residue DNA Library (SEQ ID NO: 18)
GAGATATAAATATG$(NNB)_9$TAGCCGACCGGCACCGGC
13-residue DNA Library (SEQ ID NO: 19)
GAGATATAAATATG$(NNB)_{11}$TAGCCGACCGGCACCGGC
Synthetic DNA oligo 1 (SEQ ID NO: 20)
GTAATACGACTCACTATAGGGTTAACTTTAATAAGGAGATATAAATATG
7-residue DNA Library (SEQ ID NO: 21)

GTAATACGACTCACTATAGGGTTAACTTTAATAAGGAGATATAAATATG$(NNB)_5$TAG
CCGACCGGCACCGGCACCGGCAAAAAAA

9-residue DNA Library (SEQ ID NO: 22)

GTAATACGACTCACTATAGGGTTAACTTTAATAAGGAGATATAAATATG$(NNB)_7$TAG
CCGACCGGCACCGGCACCGGCAAAAAAA

11-residue DNA Library (SEQ ID NO: 23)

GTAATACGACTCACTATAGGGTTAACTTTAATAAGGAGATATAAATATG$(NNB)_9$TAG
CCGACCGGCACCGGCACCGGCAAAAAAA

13-residue DNA Library (SEQ ID NO: 24)

GTAATACGACTCACTATAGGGTTAACTTTAATAAGGAGATATAAATATG$(NNB)_{11}$TAG
CCGACCGGCACCGGCACCGGCAAAAAAA

Example 2-2-3: Translation and cyclization of peptide display library

The translation system used was PURE system, which is a reconstituted cell-free protein synthesis system derived from a prokaryotic organism. Specifically, 1 mM GTP, 1 mM ATP, 20 mM creatine phosphate, 50 mM HEPES-KOH pH 7.6, 100 mM potassium acetate, 4 mM magnesium acetate, 2 mM spermidine, 1 mM dithiothreitol, 1 mg/mL E. coli MRE 600 (RNase-negative) derived tRNA (F. Hoffmann-La Roche, Ltd.) (some tRNAs were removed by the method described in Nucleic Acids Research, 2010, Vol. 38, No. 6 e89), 4 μg/mL creatine kinase, 6.72 U/mL myokinase, 2 units/mL inorganic pyrophosphatase, 1.1 μg/mL nucleoside diphosphate kinase, 0.26 μM EF-G, 2.7 μM IF1, 0.4 μM IF2, 1.5 μM IF3, 40 μM EF-Tu, 59 μM EF-Ts, 1.2 μM ribosome, 1 μM GlyRS, 0.04 μM IleRS, 0.16 μM ProRS, 0.09 μM ThrRS, 0.11 μM LysRS, 0.5 μM variant PheRS (WO 2016/148044), 1.37 μM AlaRS, 1 μM variant ValRS (WO 2016/148044), 1 μM variant SerRS (WO 2016/148044), 3 μM in vitro transcribed E. coli tRNA AlaIB, 250 μM glycine, 10 μM isoleucine, 250 μM proline, 250 μM threonine, 250 μM lysine, 5 mM N-methylphenylalanine, 5 mM N-methylvaline, 5 mM N-methylserine, 2.5 mM N-methylalanine, Elongator aminoacylated tRNA mixture, 25 μM Initiator aminoacylated tRNA (WO 2020/138336 A1),

0.5 μM Penicillin G Amidase (PGA), 1 μM EF-P-Lys, 0.4 units/μL RNasin (R) Ribonuclease Inhibitor (Promega Corporation), and mRNA-puromycin linked construct prepared according to WO 2013/100132 from the double-stranded DNA library described above were added to the translation reaction mixture to be 1 μM, then the mixture was left still at 37°C for 1 hour to perform the translation.

Example 2-3: Panning

Panning was performed using the translated display library for five rounds in accordance with WO 2013/100132.

Example 2-3-1: Reverse transcription of display library

In panning with the Split & Pool technique, RT primer 5 (SEQ ID NO: 25), RT primer 2 (SEQ ID NO: 5), RT primer 6 (SEQ ID NO: 26), and RT primer 7 (SEQ ID NO: 27) were used as reverse transcription primers for display libraries which were translated, cyclized, desulfurized, and purified with initial libraries with NNB of 7 residues, NNB of 9 residues, NNB of 11 residues, and NNB of 13 residues, respectively. Specifically, a 3 μM reverse transcription primer corresponding to the type of PURE, 50 mM TrisHCl (pH 8.3), 75 mM KCl, 3 mM $MgCl_2$, 0.5 mM dGTP, 0.5 mM dATP, 0.5 mM dCTP, 0.5 mM dTTP, and 8 U/μL M-MLV Reverse transcriptase (H-) (Promega Corporation, catalog# M368B) were added to 24 μL display library solution, which was translated, cyclized, desulfurized, and purified, and made up to 30 μL solution with Nuclease free water, and kept at 42°C for 60 minutes to perform the reverse transcription reaction. To this reverse transcription solution, a buffer of 1 x TBS and 2 mg/mL BSA (invitrogen) was added to prepare a 0.25 μM display library. Subsequently, in Round 1, the four types of display libraries were mixed in a ratio of 7 residues : 9 residues : 11 residues : 13 residues = 1 : 1 : 10 : 10 to form one display library solution. At the second round and thereafter, they were mixed in equal amounts for use.

RT primer 5 (SEQ ID NO: 25)
AAACACGTGGCAAACATTCCAAACCGGAGCCATACAGTACTTTTTTTgccggtgccggtg
RT primer 6 (SEQ ID NO: 26)
AAACACGTGGCAAACATTCCAACGATGATGCTCACTCTCGTTTTTTTgccggtgccggtg
RT primer 7 (SEQ ID NO: 27)
AAACACGTGGCAAACATTCCAAGACGATCCGAGCCATTACTTTTTTTgccggtgccggtg

In the panning under comparison conditions, a mixture in a ratio of 7 residues : 9 residues : 11 residues : 13 residues = 1 : 1 : 10 : 10 was prepared as an initial library, and to a 24 μL display library solution, which was translated, cyclized, desulfurized, and purified with the initial library, 3 μM Rv primer 1-1 (SEQ ID NO: 3), 50 mM TrisHCl (pH 8.3), 75 mM KCl, 3 mM $MgCl_2$, 0.5 mM dGTP, 0.5 mM dATP, 0.5 mM dCTP, 0.5 mM dTTP, and 8 U/μL M-MLV Reverse transcriptase (H-) (Promega Corporation, catalog# M368B) were added, and made up to 30 μL solution with Nuclease free water, and kept at 42°C for 60 minutes to perform the reverse transcription reaction. To this reverse transcription solution, a buffer of 1 x TBS and 2 mg/mL BSA (invitrogen) was added to prepare a 0.25 μM display library.

Example 2-3-2 Panning

In Rounds 1 to 4, GST-HisTev-Bio was fixed on Streptavidin coated magnetic beads to be 0.4 μM, and then separately added to four types of display libraries, and reacted at 4°C for 1 hour. After the reaction, the beads were magnetically collected, subjected to supernatant removal, and washed several times with tTBS (manufactured by NACALAI TESQUE, INC.), and then the beads were treated with TEV protease that recognizes and cleaves the TEV protease recognition sequence to elute the nucleic acid.

Specifically, 1 x TEV buffer and 10 mM DTT, 0.1 U/μL AcTEV protease (manufactured by Thermo Fisher Scientific, Inc., #12575015) were added as a TEV eluate to the washed beads, and reacted. After the reaction, the supernatant was recovered.

In panning with the Split & Pool technique, PCR was performed with library recognition primers (Fw primer (SEQ ID NO: 8), Rv primer 2 (SEQ ID NO: 11)).

The PCR product was subjected to PCR with the Fw primer (SEQ ID NO: 8) and Rv primer 7 (SEQ ID NO: 28), Rv primer 4 (SEQ ID NO: 13), Rv primer 8 (SEQ ID NO: 29) or Rv primer 9 (SEQ ID NO: 30) to obtain four types of PCR products derived from each library.

Rv primer 7 (SEQ ID NO: 28)
AAACCGGAGCCATACAGTAC
Rv primer 8 (SEQ ID NO: 29)
AACGATGATGCTCACTCTCG

Rv primer 9 (SEQ ID NO: 30)
AAGACGATCCGAGCCATTAC

After that, the four types of PCR products were independently subjected to PCR with primers (Fw primer (SEQ ID NO: 8), Rv primer 1-2 (SEQ ID NO: 9)) to obtain input libraries for the next round. The operations at the next round and thereafter were repeated as ones derived from Rv primer 7, Rv primer 4, Rv primer 8, and Rv primer 9 for those derived from 7 residue, 9 residue, 11 residue, and 13 residue-initial DNA libraries, respectively.

In addition, qPCR was performed using a portion of the TEV elution product, and the recovery rate of each library for each round was evaluated. For the primers of qPCR, the aforementioned primers (Fw primer (SEQ ID NO: 8) and Rv primer 7 (SEQ ID NO: 28), Rv primer 4 (SEQ ID NO: 13), Rv primer 8 (SEQ ID NO: 29) or Rv primer 9 (SEQ ID NO: 30)) were used. For the qPCR reaction solution, 1 x Ex taq buffer, 0.2 mM dNTP, 0.5 μM Fw primer, 0.5 μM Rv primer, 200,000 times dilution SYBR Green I (manufactured by Lonza K.K., catalog# 50513), and 0.02 U/μL Ex Taq polymerase (manufactured by Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

In panning with the mixed library, PCR was performed with library recognition primers (Fw primer (SEQ ID NO: 8), Rv primer 1-2 (SEQ ID NO: 9)) to obtain an input library for the next round.

In addition, qPCR was performed using a portion of the TEV elution product, and the recovery rate of the library for each round was evaluated. For the primers of qPCR, the aforementioned primers (Fw primer (SEQ ID NO: 8), Rv primer 1-1 (SEQ ID NO: 3)) were used. For the qPCR reaction solution, 1 x Ex taq buffer, 0.2 mM dNTP, 0.5 μM Fw primer, 0.5 μM Rv primer, 200,000 times dilution SYBR Green I (manufactured by Lonza K.K., catalog# 50513), and 0.02 U/μL Ex Taq polymerase (manufactured by Takara Bio Inc.) were mixed, and PCR was performed by heating at 95°C for 2 minutes, and then repeating the cycles of 95°C for 10 seconds, 57°C for 20 seconds, and 72°C for 30 seconds for 40 cycles.

To draw a calibration curve to estimate the recovery rate of the library, the input library was diluted to be 1E + 8/μL, 1E + 6/μL, and 1E + 4/μL, and subjected to qPCR under the same conditions.

At the second round and thereafter, the input library was divided into two, and selection was made with one in the presence of the target protein and the other in the absence of the target protein, and the nucleic acid recovery rates of both were evaluated from the results of qPCR. The recovery rate (%) of each round of panning was calculated by the expression below. The results are shown in Table 3.

Recovery rate of library (%) = (output nucleic acid amount (number of molecules) of library/input nucleic acid amount (number of molecules) of library) $\times$ 100

[Table 3]

| **Round** | **Panning initial library** | Input nucleic acid amount (molecules) of library | In presence of target protein | | In absence of target protein | |
|---|---|---|---|---|---|---|
| | | | Output nucleic acid amount (molecules) | Recovery rate (%) | Output nucleic acid amount (molecules) | Recovery rate (%) |
| **1** | 7-residue | **3.33.E+10** | **1.08.E+04** | **3.23.E-05** | **-** | **-** |
| **2** | | **1.06.E+11** | **1.55.E+06** | **1.46.E-03** | **3.29.E+05** | **3.10.E-04** |
| **3** | | **1.59.E+11** | **2.03.E+07** | **1.28.E-02** | **1.89.E+06** | **1.19.E-03** |
| **4** | | **7.17.E+10** | **1.68.E+08** | **2.35.E-01** | **6.40.E+05** | **8.93.E-04** |
| **1** | 9-residue | **4.62.E+10** | **2.17.E+04** | **4.70.E-05** | **-** | **-** |
| **2** | | **1.25.E+11** | **1.38.E+06** | **1.10.E-03** | **8.28.E+05** | **6.62.E-04** |
| **3** | | **3.10.E+11** | **6.00.E+07** | **1.94.E-02** | **5.04.E+06** | **1.63.E-03** |
| **4** | | **7.85.E+10** | **1.91.E+08** | **2.44.E-01** | **3.62.E+05** | **4.61.E-04** |
| **1** | 11-residue | **4.13.E+11** | **1.55.E+05** | **3.76.E-05** | **-** | **-** |
| **2** | | **9.86.E+10** | **1.15.E+06** | **1.16.E-03** | **3.52.E+05** | **3.57.E-04** |
| **3** | | **1.30.E+11** | **3.86.E+08** | **2.97.E-01** | **2.58.E+06** | **1.98.E-03** |
| **4** | | **8.43.E+10** | **1.36.E+09** | **1.62.E+00** | **3.97.E+05** | **4.71.E-04** |

(continued)

| Round | Panning initial library | Input nucleic acid amount (molecules) of library | In presence of target protein | | In absence of target protein | |
|---|---|---|---|---|---|---|
| | | | Output nucleic acid amount (molecules) | Recovery rate (%) | Output nucleic acid amount (molecules) | Recovery rate (%) |
| **1** | 13-residue | **4.52.E+11** | **1.06.E+05** | **2.34.E-05** | **-** | **-** |
| **2** | | **1.23.E+11** | **1.41.E+06** | **1.14.E-03** | **5.04.E+05** | **4.10.E-04** |
| **3** | | **2.31.E+11** | **1.76.E+07** | **7.64.E-03** | **4.12.E+06** | **1.78.E-03** |
| **4** | | **6.73.E+10** | **1.86.E+08** | **2.77.E-01** | **4.60.E+05** | **6.84.E-04** |
| **1** | **7,9,11,13** -residue mixture | **4.29.E+12** | **3.19.E+07** | **7.45.E-04** | **-** | **-** |
| **2** | | **8.27.E+11** | **3.90.E+06** | **4.71.E-04** | **1.21.E+06** | **1.47.E-04** |
| **3** | | **1.06.E+12** | **9.08.E+08** | **8.57.E-02** | **2.23.E+07** | **2.11.E-03** |
| **4** | | **7.47.E+11** | **1.58.E+10** | **2.12.E+00** | **2.32.E+06** | **3.11.E-04** |

As a result, in the second half of the rounds, as shown in Table 3, there was a difference in the recovery rate between the selections in the presence of the target protein and in the absence of the target protein. It is thus likely that molecules that are only recovered in the presence of the target protein are present in the library of each round, and they are enriched each time the round is repeated.

<u>Example 2-3-3 Analysis of enrichment sequence</u>

The base sequences of the DNA pools from each round of panning were analyzed. A sequence in which a value obtained by setting the sequences differing only by one amino acid residue to the same cluster and dividing the maximum number of NGS reads of each sequence by the maximum number of NGS reads of the sequence with the largest number of reads in the same cluster was greater than 0.01, and in which the frequency of occurrence was more than 0.05% and the frequency of occurrence increased 10 times or more when a target was added in at least one round compared to when the pool of the previous round was divided and the target was not added in the panning was extracted as a sequence enriched only in the presence of target protein except for sequences that appeared due to NGS, PCR errors.

The number of sequences extracted is shown in Table 4 for each panning result. In Table 4, "AA" represents amino acid residues, and "7AA," "9AA," "11AA," and "13AA" represent sequences appearing in the 7-residue initial library, the 9-residue initial library, the 11-residue initial library, and the 13-residue initial library, respectively.

[Table 4]

| Panning initial library | Number of enrichment sequences in presence of target protein | | | |
|---|---|---|---|---|
| | **7AA** | **9AA** | **11AA** | **13AA** |
| 7-residue | **15** | **-** | **2** | **-** |
| 9-residue | **-** | **17** | **2** | **-** |
| 11-residue | **-** | **-** | **12** | **-** |
| 13-residue | **-** | **-** | **2** | **5** |
| 7,9,11,13-residue mixture | **2** | **5** | **12** | **1** |

As a result, as shown in Table 4, the sequences derived from 11-residue initial library was significantly enriched but the sequences derived from other initial libraries were few in the panning with the mixed library, while in the panning using Split & Pool, even sequences derived from 7, 9, and 13-residue initial libraries, which were difficult to enrich with the mixed library, could be found in large numbers. Thus, a variety of candidate binder sequences could be found with the Split & Pool panning.

[Sequence Listing]

## Claims

1. A method for screening for a candidate peptide capable of binding to a target molecule, the method comprising the steps of:

   (1) preparing a plurality of nucleic acid display libraries containing a barcoded peptide-nucleic acid complex, wherein the barcoded peptide-nucleic acid complex contains a nucleic acid moiety and a peptide moiety, the nucleic acid moiety contains a barcode sequence and a nucleic acid sequence encoding the peptide, and the plurality of nucleic acid display libraries are nucleic acid display libraries, each of which is independently produced by translation using a cell-free translation system;
   (2) mixing the plurality of nucleic acid display libraries to prepare a mixed nucleic acid display library;
   (3) bringing the mixed nucleic acid display library into contact with the target molecule; and
   (4) amplifying a nucleic acid corresponding to the nucleic acid moiety of the barcoded peptide-nucleic acid complex bound to the target molecule, using a barcode primer.

2. The method according to claim 1, wherein the step (1) includes translating a nucleic acid encoding a peptide to prepare a peptide and linking the peptide to the nucleic acid to prepare a peptide-nucleic acid complex.

3. The method according to claim 1 or 2, wherein the step (1) includes barcoding the peptide-nucleic acid complex to prepare a barcoded peptide-nucleic acid complex.

4. The method according to any one of claims 1 to 3, wherein the barcoding is reverse transcribing the nucleic acid moiety of the peptide-nucleic acid complex.

5. The method according to any one of claims 1 to 4, wherein the barcoding is reverse transcribing the nucleic acid moiety of the peptide-nucleic acid complex with a first primer containing a barcode sequence.

6. The method according to any one of claims 1 to 5, wherein the plurality of nucleic acid display libraries have different barcode sequences for each nucleic acid display library.

7. The method according to any one of claims 1 to 6, wherein the plurality of nucleic acid display libraries are nucleic acid display libraries, each of which is independently produced by translation using a different cell-free translation system from one another.

8. The method according to any one of claims 1 to 7, wherein in the step (2), two or more nucleic acid display libraries are mixed.

9. The method according to any one of claims 1 to 8, comprising, between the steps (3) and (4), a step (3A) of eluting the nucleic acid moiety from the barcoded peptide-nucleic acid complex bound to the target molecule.

10. The method according to any one of claims 1 to 9, further comprising, between the steps (3) and (4), a step (3B) of identifying an amino acid sequence of the peptide moiety of the barcoded peptide-nucleic acid complex bound to the target molecule.

11. The method according to any one of claims 1 to 10, wherein in the step (4), a nucleic acid of the barcoded peptide-nucleic acid complex having the same barcode sequence as a barcode sequence of the barcode primer is selectively amplified.

12. The method according to any one of claims 1 to 11, wherein the barcode primer is a second primer containing a barcode sequence.

13. The method according to any one of claims 1 to 12, wherein the steps (1) to (4) constitute a cycle, and the cycle is repeated multiple times.

14. The method according to any one of claims 1 to 13, further comprising, after the step (4) or before starting a next cycle, a step (5) of further amplifying the nucleic acid amplified in the step (4) with a primer that does not contain a barcode sequence.

**15.** The method according to any one of claims 1 to 14, wherein the nucleic acid display library is a library having a diversity of $10^4$ or more.

*Fig.1*

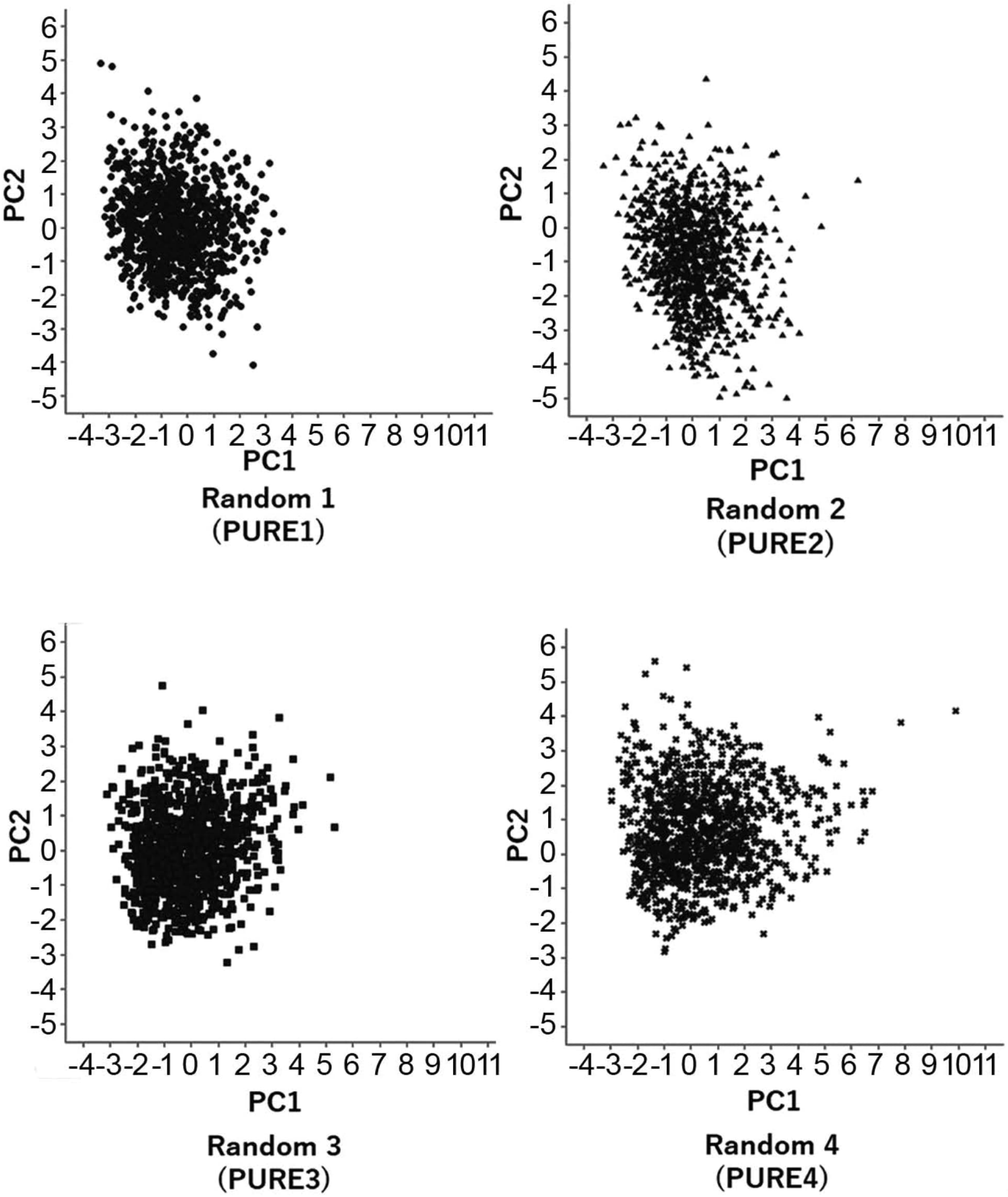
PC2
PC1
Random 1
(PURE1)
Random 2
(PURE2)
Random 3
(PURE3)
Random 4
(PURE4)

*Fig.2*

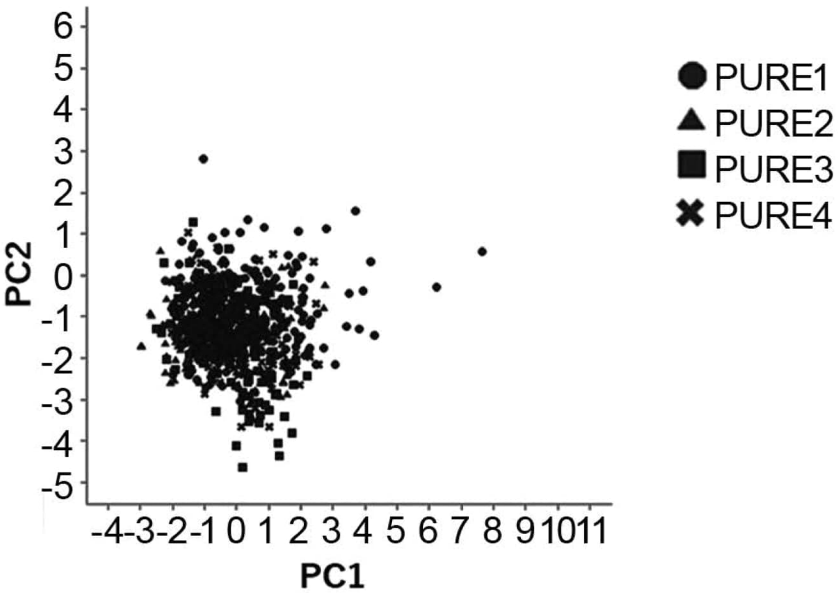
PURE1
PURE2
PURE3
PURE4
PC2
PC1
6
5
4
3
2
1
0
-1
-2
-3
-4
-5
-4-3-2-1 0 1 2 3 4 5 6 7 8 9 10 11

*Fig.3*

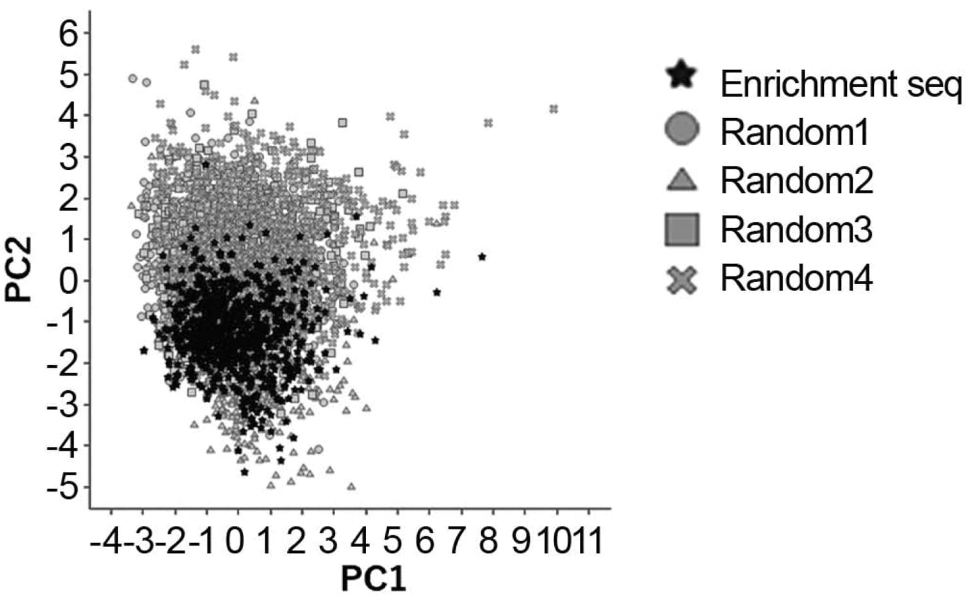
6
5
4
3
2
1
0
-1
-2
-3
-4
-5
PC2
-4-3-2-1 0 1 2 3 4 5 6 7 8 9 10 11
PC1
Enrichment seq
Random1
Random2
Random3
Random4

## INTERNATIONAL SEARCH REPORT

International application No.
**PCT/JP2024/036319**

**A. CLASSIFICATION OF SUBJECT MATTER**

***C12Q 1/6844***(2018.01)i; ***C40B 40/02***(2006.01)i
FI: C12Q1/6844 Z; C40B40/02

According to International Patent Classification (IPC) or to both national classification and IPC

**B. FIELDS SEARCHED**

Minimum documentation searched (classification system followed by classification symbols)

C12Q1/6844; C40B40/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | HENSON, S. N. et al. PepSeq: a fully in vitro platform for highly multiplexed serology using customizable DNA-barcoded peptide libraries. NATURE PROTOCOLS. 2022, vol. 18, pp. 396-423<br>entire text, in particular, abstract, fig. 1 | 1-15 |
| A | JP 2022-126865 A (ENCODIA INC.) 30 August 2022 (2022-08-30)<br>entire text | 1-15 |
| A | JP 2022-513092 A (NAUTILUS BIOTECHNOLOGY, INC.) 07 February 2022 (2022-02-07)<br>entire text | 1-15 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 November 2024** | **19 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No.
**PCT/JP2024/036319**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/036319**

| Patent document cited in search report | Publication date (day/month/year) | Patent family member(s) | Publication date (day/month/year) |
|---|---|---|---|
| JP 2022-126865 A | 30 August 2022 | WO 2017/192633 A1<br>entire text | |
| JP 2022-513092 A | 07 February 2022 | WO 2020/106889 A1<br>entire text | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2022138892 A
- WO 2018143145 A
- WO 2018225864 A
- WO 2020138336 A1
- WO 2016148044 A


### Non-patent literature cited in the description


- **D.E. HACKER et al.** *ACS Chem. Biol.*, 2017, vol. 12, 795-804
- **M.E. BROUSSEAU et al.** *Cell Chem. Biol.*, 2022, vol. 29, 249-258
- *Proc Natl Acad Sci USA*, 1997, vol. 94, 12297-302
- *FEBS Lett*, 1997, vol. 414, 405-8
- *J Am Chem Soc.*, 15 April 2009, vol. 131 (14), 5040-1
- *Biochemistry*, 2003, vol. 42, 4787-99
- **KAWAKAMI T et al.** Ribosomal synthesis of polypeptoids and peptoid-peptide hybrids. *J Am Chem Soc.*, 2008, vol. 130, 16861-3
- **KAWAKAMI T et al.** Diverse backbone-cyclized peptides via codon reprogramming. *Nat Chem Biol*, 2009, vol. 5, 888-90
- **DEDKOVA LM et al.** Construction of modified ribosomes for incorporation of D-amino acids into proteins. *Biochemistry*, 2006, vol. 45, 15541-51
- **DOI Y et al.** Elongation factor Tu mutants expand amino acid tolerance of protein biosynthesis system. *J Am Chem Soc.*, 2007, vol. 129, 14458-62
- **PARK HS et al.** Expanding the genetic code of Escherichia coli with phosphoserine. *Science*, 2011, vol. 333, 1151-4
- **CHEN HZ** ; **ZUBAY G.** Prokaryotic coupled transcription-translation. *Methods Enzymol.*, 1983, vol. 101, 674-90
- **E GASIOR** ; **F HERRERA** ; **I SADNIK** ; **C S MCLAUGHLIN** ; **K MOLDAVE**. The preparation and characterization of a cell-free system from Saccharomyces cerevisiae that translates natural messenger ribonucleic acid. *J. Biol. Chem.*, 1979, vol. 254, 3965-3969
- **ERICKSON AH** ; **BLOBEL G.** Cell-free translation of messenger RNA in a wheat germ system. *Methods Enzymol*, 1983, vol. 96, 38-50
- **JACKSON RJ** ; **HUNT T.** Preparation and use of nuclease-treated rabbit reticulocyte lysates for the translation of eukaryotic messenger RNA. *Methods Enzymol*, 1983, vol. 96, 50-74
- **BARTON DJ** ; **MORASCO BJ** ; **FLANEGAN JB**. Assays for poliovirus polymerase, 3D(Pol), and authentic RNA replication in HeLa S10 extracts. *Methods Enzymol.*, 1996, vol. 275, 35-57
- **SWERDEL MR** ; **FALLON AM**. Cell-free translation in lysates from Spodoptera frugiperda (Lepidoptera:Noctuidae) cells. *Comp Biochem Physiol B.*, 1989, vol. 93, 803-6
- **SHIMIZU Y** ; **INOUE A** ; **TOMARI Y** ; **SUZUKI T** ; **YOKOGAWA T** ; **NISHIKAWA K** ; **UEDA T**. Cell-free translation reconstituted with purified components. *Nat Biotechnol.*, 2001, vol. 19, 751-5
- **SHIMIZU Y** ; **UEDA T**. PURE technology. *Methods Mol Biol*, 2010, vol. 607, 11-21
- **ANDERSON JC** ; **SCHULTZ PG.** Adaptation of an orthogonal archaeal leucyl-tRNA and synthetase pair for four-base, amber, and opal suppression. *Biochemistry*, 2003, vol. 42, 9598-608
- **MURAKAMI H** ; **KOUROUKLIS D** ; **SUGA H**. Using a solid-phase ribozyme aminoacylation system to reprogram the genetic code. *Chem Biol*, 2003, vol. 10, 1077-84
- **KIGA D** ; **SAKAMOTO K** ; **KODAMA K** ; **KIGAWA T** ; **MATSUDA T** ; **YABUKI T** ; **SHIROUZU M** ; **HARADA Y** ; **NAKAYAMA H** ; **TAKIO K**. An engineered Escherichia coli tyrosyl-tRNA synthetase for site-specific incorporation of an unnatural amino acid into proteins in eukaryotic translation and its application in a wheat germ cell-free system. *Proc. Natl. Acad. Sci. U S A.*, 2002, vol. 99, 9715-20
- **CHIN JW** ; **CROPP TA** ; **ANDERSON JC** ; **MUKHERJI M** ; **ZHANG Z** ; **SCHULTZ PG** ; **CHIN,JW.** An expanded eukaryotic genetic code. *Science*, 2003, vol. 301, 964-7

- **HARTMAN MC** ; **JOSEPHSON K** ; **SZOSTAK JW**. Enzymatic aminoacylation of tRNA with unnatural amino acids. *Proc. Natl. Acad. Sci. U S A.*, 2006, vol. 103, 4356-61
- **SUBTELNY AO** ; **HARTMAN MC** ; **SZOSTAK JW**. Ribosomal synthesis of N-methyl peptides. *J Am Chem Soc.*, 2008, vol. 130, 6131-6
- **HECKLER TG** ; **CHANG LH** ; **ZAMA Y** ; **NAKA T** ; **CHORGHADE MS** ; **HECHT SM**. T4 RNA ligase mediated preparation of novel ''chemically misacylated'' tRNAPheS. *Biochemistry*, 1984, vol. 23, 1468-73
- **MURAKAMI H** ; **BONZAGNI NJ** ; **SUGA H**. Aminoacyl-tRNA synthesis by a resin-immobilized ribozyme. *J Am Chem Soc*, 2002, vol. 124, 6834-5
- **HASHIMOTO N** ; **NINOMIYA K** ; **ENDO T** ; **SISIDO M**. Simple and quick chemical aminoacylation of tRNA in cationic micellar solution under ultrasonic agitation. *Chem Commun (Camb)*, 2005 (34), 4321-3
- **NINOMIYA K** ; **MINOHATA T** ; **NISHIMURA M** ; **SISIDO M**. In situ chemical aminoacylation with amino acid thioesters linked to a peptide nucleic acid. *J Am Chem Soc.*, 2004, vol. 126, 15984-9
- **JOSEPHSON K** ; **HARTMAN MC** ; **SZOSTAK JW**. Ribosomal synthesis of unnatural peptides. *J. Am. Chem. Soc.*, 2005, vol. 127, 11727-35
- **KWON I et al.** Breaking the degeneracy of the genetic code. *J Am Chem Soc*, 2003, vol. 125, 7512-3
- **YAMAGUCHI J** ; **NAIMUDDIN M** ; **BIYANI M** ; **SASAKI T** ; **MACHIDA M** ; **KUBO T** ; **FUNATSU T** ; **HUSIMI Y** ; **NEMOTO N**. cDNA display: a novel screening method for functional disulfide-rich peptides by solid-phase synthesis and stabilization of mRNA-protein fusions. *Nucleic Acids Res*, 2009, vol. 37 (16), e108
- **MATTHEAKIS LC** ; **BHATT RR** ; **DOWER WJ**. An in vitro polysome display system for identifying ligands from very large peptide libraries. *Proc Natl Acad Sci U S A*, 1994, vol. 91, 9022-6
- **REIERSEN H** ; **LOBERSLI I** ; **LOSET GA** ; **HVATTUM E** ; **SIMONSEN B** ; **STACY JE** ; **MCGREGOR D** ; **FITZGERALD K** ; **WELSCHOF M** ; **BREKKE OH**. Covalent antibody display--an in vitro antibody-DNA library selection system. *Nucleic Acids Res*, 2005, vol. 33, e10
- **ODEGRIP R** ; **COOMBER D** ; **ELDRIDGE B** ; **HEDERER R** ; **KUHLMAN PA** ; **ULLMAN C** ; **FITZGERALD K** ; **MCGREGOR D**. CIS display: In vitro selection of peptides from libraries of protein-DNA complexes. *Proc Natl Acad Sci U S A*, 2004, vol. 101, 2806-10
- **TAWFIK DS** ; **GRIFFITHS AD**. Man-made cell-like compartments for molecular evolution. *Nat Biotechnol*, 1998, vol. 16, 652-6
- **DOUTHWAITE J. A. et al.** Ribosome Display and Related Technologies: Methods and Protocols. Humana, 2011, 113-135
- *Nucleic Acids Research*, 2010, vol. 38 (6), e89